# EUROPEAN PATENT SPECIFICATION

(11) **EP 2 155 254 B1**
(45) Date of publication and mention of the grant of the patent: **28.11.2012**
(21) Application number: 08769347.9
(22) Date of filing: 08.05.2008
(51) Int. Cl.: A61K 47/48, A61K 49/08, A61K 49/00, A61K 49/14, A61P 35/00

(54) **POLYMERS CONJUGATED WITH PLATINUM DRUGS**
MIT PLATINARZNEIMITTELN KONJUGIERTE POLYMERE
POLYMÈRES CONJUGUÉS AVEC DES MÉDICAMENTS À BASE DE PLATINE

(30) Priority: 09.05.2007 US 916857 P
(43) Date of publication of application: 24.02.2010
(73) Proprietor: NITTO DENKO CORPORATION, Osaka 567-8680 (JP)
(72) Inventor: VAN, Sang, San Diego, CA 92111 (US); ZHAO, Gang, Vista, CA 92081 (US); YU, Lei, Carlsbad, CA 92008 (US)
(74) Representative: Miles, John Stephen
(86) International application number: PCT/US2008/063128
(87) International publication number: WO 2008/141111

(56) References cited:
- EP-A- 1 695 991
- WO-A-03/017923
- YE HAIFENG ET AL: "Poly([gamma],l-glutamic acid)-cisplatin conjugate effectively inhibits human breast tumor xenografted in nude mice" BIOMATERIALS 200612 GB, vol. 27, no. 35, December 2006 (2006-12), pages 5958-5965, XP002522733 ISSN: 0142-9612

## Description

### BACKGROUND OF THE INVENTION

### Field of the Invention

Generally disclosed herein are biocompatible polymers having platinum compounds conjugated thereto and methods for making them. The polymer conjugates described herein are useful for a variety of drug, biomolecule, and imaging agent delivery applications. Also disclosed are the polymer conjugates described herein for use to treat, diagnose, and/or image a subject.

### Description of the Related Art

A variety of systems have been used for the delivery of drugs, biomolecules, and imaging agents. For example, such systems include capsules, liposomes, microparticles, nanoparticles, and polymers.

A variety of polyester-based biodegradable systems have been characterized and studied. Polylactic acid (PLA), polyglycolic acid and their copolymers polylactic-co-glycolic acid (PLGA) are some of the better characterized biomaterials with regard to design and performance for drug-delivery applications. See Uhrich, K.E.; Cannizzaro, S.M.; Langer, R.S. and Shakeshelf, K.M. "Polymeric Systems for Controlled Drug Release," Chem. Rev. 1999, 99, 3181-3198 and Panyam J, Labhasetwar V. "Biodegradable nanoparticles for drug and gene delivery to cells and tissue," Adv. Drug. Deliv. Rev. 2003, 55, 329-47. Also, 2-hydroxypropyl methacrylate (HPMA) has been widely used to create a polymer for drug-delivery applications. Biodegradable systems based on polyorthoesters have also been investigated. See Heller, J.; Barr, J.; Ng, S.Y.; Abdellauoi, K.S. and Gurny, R. "Poly(ortho esters): synthesis, characterization, properties and uses." Adv. Drug Del. Rev. 2002, 54, 1015-1039. Polyanhydride systems have also been investigated. Such polyanhydrides are typically biocompatible and may degrade in vivo into relatively non-toxic compounds that are eliminated from the body as metabolites. See Kumar, N.; Langer, R.S. and Domb, A.J. "Polyanhydrides: an overview," Adv. Drug Del. Rev. 2002, 54, 889-91.

Amino acid-based polymers have also been considered as a potential source of new biomaterials. Poly-amino acids having good biocompatibility have been investigated to deliver low molecular-weight compounds. A relatively small number of polyglutamic acids and copolymers have been identified as candidate materials for drug delivery. See Bourke, S.L. and Kohn, J. "Polymers derived from the amino acid L-tyrosine: polycarbonates, polyarylates and copolymers with poly(ethylene glycol)." Adv. Drug Del. Rev., 2003, 55, 447-466.

Administered hydrophobic anticancer drugs and therapeutic proteins and polypeptides often suffer from poor bio-availability. Such poor bio-availability may be due to incompatibility of bi-phasic solutions of hydrophobic drugs and aqueous solutions and/or rapid removal of these molecules from blood circulation by enzymatic degradation. One technique for increasing the efficacy of administered proteins and other small molecule agents entails conjugating the administered agent with a polymer, such as a polyethylene glycol ("PEG") molecule, that can provide protection from enzymatic degradation in vivo. Such "PEGylation" often improves the circulation time and, hence, bio-availability of an administered agent.

PEG has shortcomings in certain respects, however. For example, because PEG is a linear polymer, the steric protection afforded by PEG is limited, as compared to branched polymers. Another shortcoming of PEG is that it is generally amenable to derivatization at its two terminals. This limits the number of other functional molecules (e.g. those helpful for protein or drug delivery to specific tissues) that can be conjugated to PEG.

Polyglutamic acid (PGA) is another polymer of choice for solubilizing hydrophobic anticancer drugs. Many anti-cancer drugs conjugated to PGA have been reported. See Chun Li. "Poly(L-glutamic acid)-anticancer drug conjugates." Adv. Drug Del. Rev., 2002, 54, 695-713. However, none are currently FDA-approved.

Paclitaxel, extracted from the bark of the Pacific Yew tree, is a FDA-approved drug for the treatment of ovarian cancer and breast cancer. Wani et al. "Plant antitumor agents. VI. The isolation and structure of taxol, a novel antileukemic and antitumor agent from Taxus brevifolia," J. Am. Chem. Soc. 1971, 93, 2325-7. However, like other anti-cancer drugs, pacilitaxel suffers from poor bio-availability due to its hydrophobicity and insolubility in aqueous solution. One way to solubilize pacilitaxel is to formulate it in a mixture of Cremophor-EL and dehydrated ethanol (1:1, v/v). Sparreboom et al. "Cremophor EL-mediated Alteration of Paclitaxel Distribution in Human Blood: Clinical Pharmacokinetic Implications," Cancer Research, 1999, 59, 1454-1457. This formulation is currently commercialized as Taxol^{®} (Bristol-Myers Squibb). Another method of solubilizing paclitaxel is by emulsification using high-shear homogenization. Constantinides et al. "Formulation Development and Antitumor Activity of a Filter-Sterilizable Emulsion of Paclitaxel," Pharmaceutical Research 2000, 17, 175-182. Recently, polymer-paclitaxel conjugates have been advanced in several clinical trials. Ruth Duncan "The Dawning era of polymer therapeutics," Nature Reviews Drug Discovery 2003, 2, 347-360. More recently, paclitaxel has been formulated into nano-particles with human albumin protein and has been used in clinical studies. Damascelli et al. "Intraarterial chemotherapy with polyoxyethylated castor oil free paclitaxel, incorporated in albumin nanoparticles (ABI-007): Phase II study of patients with squamous cell carcinoma of the head and neck and anal canal: preliminary evidence of clinical activity." Cancer, 2001, 92, 2592-602, and Ibrahim et al. "Phase I and pharmacokinetic study of ABI-007, a Cremophor-free, protein-stabilized, nanoparticle formulation of paclitaxel," Clin. Cancer Res. 2002, 8, 1038-44. This formulation is currently commercialized as Abraxane^{®} (American Pharmaceutical Partners, Inc.).

Hydrophilic anticancer drugs, such as platinum based-drugs, are another class of clinically-approved drugs that is effective for treating cancers. However, these drugs are still limited for use in clinics due to toxicity. Many cisplatin derivatives have been explored for therapeutics, but few have been successful. One approach is to use platinum drugs is to conjugate onto macromolecules or polymers. See U.S. Patent Nos. 5,965,118 (HPMA-oligopeptide-Pt); 6,692,734 (HPMA-oligopeptide-amidomalonate-Pt); 5,985,916 (Diamido-diamine polymer-Pt); 7,166,733 (Amidomalonate-Pt complexes); Haag et al., "Polymer therapeutics: Concept and application," Agnew. Chem. Int. End, 2006, 45, 1198-1215; Duncan, Ruth, "The Dawning Era of Polymer Therapeutics," Nature Reviews Drugs Discovery, 2003, 2, 347-360. Another approach is to use a platinum drug to incorporate it into polymeric micelles. See Nishiyama et al., "Preparation and characterization of size-controlled polymeric micelle containing cis-dichlorodiammineplatinum(II) in the core," Journal of Controlled Release, 74 (2001) 83-94; Nishiyama et al., "Novel cisplatin-incorporated polymeric micelles can eradicate solid tumors in mice," Cancer Research, 63, December 15, 2003, 8977-8983. Uchino et al., "Cisplatin-incorporating polymeric micelles (NC-6004) can reduce nephrotoxicity and neurotoxicity of cisplatin in rats," British Journal of Cancer, 93 (2005), 678-687. However, while polymer-platinum conjugates have been proposed as an approach to increasing solubility and reducing systemic toxicity, few have successfully entered clinical investigation and few have displayed significant benefit in vivo. Failure has been due to lack of biocompatibility, toxicity of the proposed carrier, lack of antitumor activity and/or other problems. Accordingly, there is still a need for finding an effective way to formulate and utilize platinum drugs for treating cancers.

WO 03/01723 describes anticancer polypeptide-metal complexes and compositions, methods of making, and methods of using the same.

### SUMMARY OF THE INVENTION

The inventors have discovered a series of novel polyglutamate-amino acids that are capable of conjugating to platinum-containing compounds, such as drugs that contain platinum. A number of agents, such as imaging agents, targeting agents and/or other drugs may also be conjugated to the polyglutamate-amino acids. In certain embodiments, the polymers and the resulting conjugates preferentially accumulate in certain tissues (e.g., tumor tissues) and/or certain receptors, and thus are useful for delivering drugs (e.g., anticancer drugs, including platinum-containing anticancer drugs) and/or imaging agents to specific parts of the body (e.g., tumors). In certain embodiments, the polymers and the resulting polymer conjugates can form nanoparticles that effectively solubilize the imaging agent, targeting agent, magnetic resonance imaging agent, and/or drug in aqueous systems by dispersing it at a molecular level, thereby increasing functionality and/or bioavailability.

The polymer conjugate of the invention comprises at least one recurring unit selected from formula (I), formula (II), and formula (III): 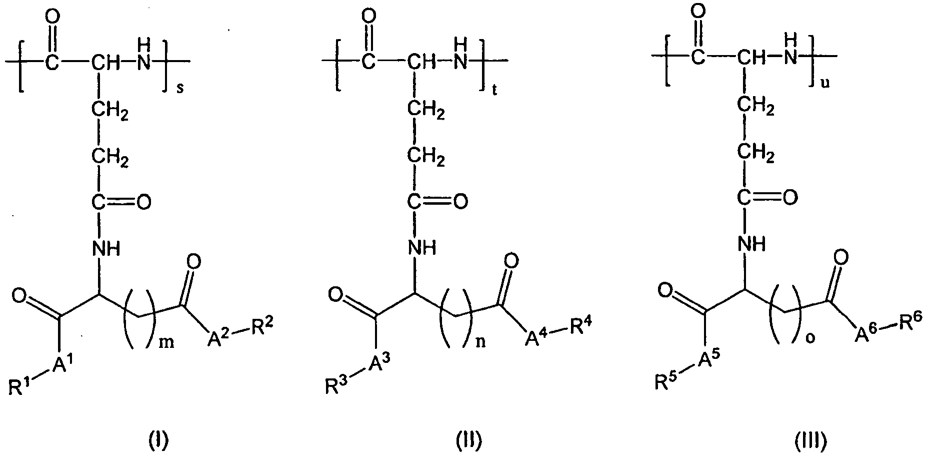 wherein:
A¹, A², A³, A⁴, A⁵ and A⁶ are each independently oxygen or NR⁷, wherein R⁷ is hydrogen or a C₁₋₄ alkyl;
R¹, R², R³, R⁴, R⁵ and R⁶ are each independently selected from the group consisting of a hydrogen, a C₁₋₁₀ alkyl group, a C₆₋₂₀ aryl group, an ammonium group, an alkali metal, a polydentate ligand, a polydentate ligand precursor with protected oxygen atoms, a group that comprises platinum, a group that comprises a drug, a group that comprises a targeting agent, a group that comprises an optical imaging agent, a group that comprises a magnetic resonance imaging agent, and a group that comprises a stabilizing agent;
wherein the polydentate ligand comprises: 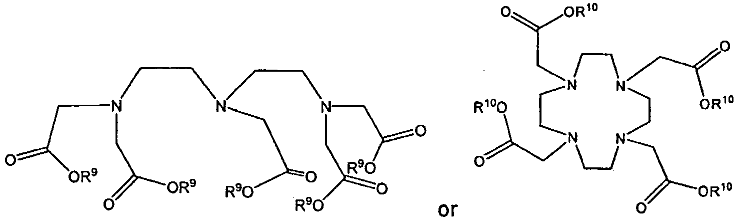 wherein each R⁹ is independently hydrogen, ammonium, or an alkali metal; and
wherein each R¹⁰ is independently hydrogen, ammonium, or an alkali metal;
wherein the polydentate ligand precursor with protected oxygen atoms comprises: 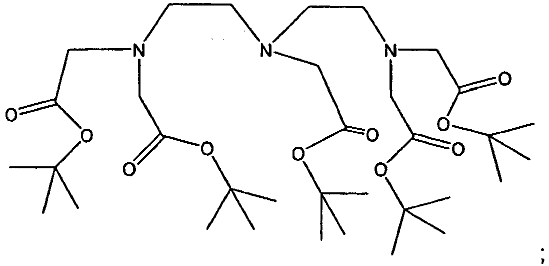 wherein the group that comprises platinum comprises cisplatin, carboplatin, or oxaliplatin, or wherein the recurring unit of formula (I) has the structure: 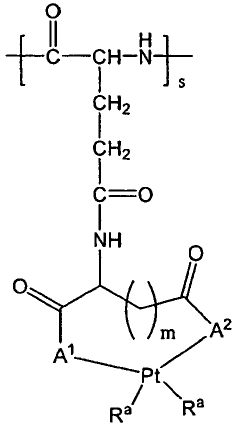 wherein each R^{a} is independently selected from the group consisting of monoalkyl amine, dialkylamine, monoaryl amine, and diaryl amine;
wherein the group that comprises a drug comprises an anticancer drug; wherein the group that comprises the targeting agent is selected from the group consisting of an arginine-glycine-aspartate (RGD) peptide, fibronectin, folate, galactose, an apolipoprotein, insulin, transferrin, a fibroblast growth factor (FGF), an epidermal growth factor (EGF), and an antibody;
wherein the group that comprises an optical imaging agent is selected from the group consisting of an acridine dye, a coumarine dye, a rhodamine dye, a xanthene dye, a cyanine dye, and a pyrene dye;
wherein the group that comprises a magnetic resonance imaging agent
comprises: 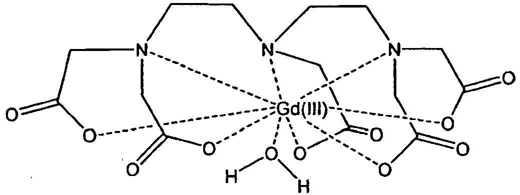 or 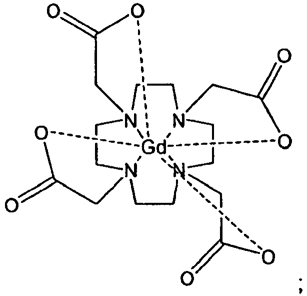 wherein the group that comprises a stabilizing agent comprises polyethylene glycol;
at least one of R¹, R², R³, R⁴, R⁵ and R⁶ is a group that comprises platinum;
m, n, and o are each independently 1 or 2; and
s, t, and u are each independently 0 or ≥ 1, wherein s+t+u is ≥ 1.

Another embodiment described herein relates to a method of making the polymer conjugate of the invention that can include dissolving or partially dissolving a polymeric reactant comprising a recurring unit of formula (V), as described herein, in a solvent to form a dissolved or partially dissolved polymeric reactant; wherein z can be independently 1 or 2; A⁷ and A⁸ can be oxygen; and R¹¹ and R¹² can be each independently selected from hydrogen, ammonium, and an alkali metal; and reacting the dissolved or partially dissolved polymeric reactant with a second reactant, wherein the second reactant comprises a group comprising platinum.

Another embodiment described herein relates to a composition that can include the polymer conjugate described herein, and further comprising at least one selected from a pharmaceutically acceptable excipient, a carrier, and a diluent.

Another embodiment described herein relates to the polymer conjugate of the invention for use in treating or ameliorating a disease or condition that can include administering an effective amount of the polymer conjugate described herein to a mammal in need thereof.

Another embodiment provides the polymer conjugate of the invention for use in diagnosing a disease or condition that can include administering an effective amount of the polymer conjugate described herein to a mammal.

Another embodiment provides the polymer conjugate of the invention for use in imaging a portion of tissue that can include contacting a portion of tissue with an effective amount of the polymer conjugate described herein.

These and other embodiments are described in greater detail below.

### DETAILED DESCRIPTION OF THE PREFERRED EMBODIMENTS

Unless defined otherwise, all technical and scientific terms used herein have the same meaning as is commonly understood by one of ordinary skill in the art. In the event that there are a plurality of definitions for a term herein, those in this section prevail unless stated otherwise.

The term "ester" is used herein in its ordinary sense, and thus includes a chemical moiety with formula -(R)ₙ-COOR', where R and R' are independently selected from the group consisting of alkyl, cycloalkyl, aryl, heteroaryl (bonded through a ring carbon) and heteroalicyclic (bonded through a ring carbon), and where n is 0 or 1.

The term "amide" is used herein in its ordinary sense, and thus includes a chemical moiety with formula -(R)ₙ-C(O)NHR' or -(R)ₙ-NHC(O)R', where R and R' are independently selected from the group consisting of alkyl, cycloalkyl, aryl, heteroaryl (bonded through a ring carbon) and heteroalicyclic (bonded through a ring carbon), and where n is 0 or 1. An amide may be included in an amino acid or a peptide molecule attached to drug molecule as described herein, thereby forming a prodrug.

Any amine, hydroxy, or carboxyl side chain on the compounds disclosed herein can be esterified or amidified. The procedures and specific groups to be used to achieve this end are known to those of skill in the art and can readily be found in reference sources such as Greene and Wuts, Protective Groups in Organic Synthesis, 3rd Ed., John Wiley & Sons, New York, NY, 1999.

As used herein, "alkyl" refers to a straight or branched hydrocarbon chain that comprises a fully saturated (no double or triple bonds) hydrocarbon group. The alkyl group may have 1 to 20 carbon atoms (whenever it appears herein, a numerical range such as "1 to 20" refers to each integer in the given range; *e.g*., "1 to 20 carbon atoms" means that the alkyl group may consist of 1 carbon atom, 2 carbon atoms, 3 carbon atoms, *etc.,* up to and including 20 carbon atoms, although the present definition also covers the occurrence of the term "alkyl" where no numerical range is designated). The alkyl group may also be a medium size alkyl having 1 to 10 carbon atoms. The alkyl group could also be a lower alkyl having 1 to 5 carbon atoms. The alkyl group of the compounds may be designated as "C₁-C₄ alkyl" or similar designations. By way of example only, "C₁-C₄ alkyl" indicates that there are one to four carbon atoms in the alkyl chain, i.e., the alkyl chain is selected from methyl, ethyl, propyl, iso-propyl, n-butyl, iso-butyl, sec-butyl, and t-butyl. Typical alkyl groups include methyl, ethyl, propyl, isopropyl, butyl, isobutyl, tertiary butyl, pentyl, hexyl.

The alkyl group may be substituted or unsubstituted. When substituted, the substituent group(s) is(are) one or more group(s) individually and independently selected from alkenyl, alkynyl, cycloalkyl, cycloalkenyl, cycloalkynyl, aryl, heteroaryl, heteroalicyclyl, aralkyl, heteroaralkyl, (heteroalicyclyl)alkyl, hydroxy, protected hydroxyl, alkoxy, aryloxy, acyl, ester, mercapto, alkylthio, arylthio, cyano, halogen, carbonyl, thiocarbonyl, O-carbamyl, N-carbamyl, O-thiocarbamyl, N-thiocarbamyl, C-amido, N-amido, S-sulfonamido, N-sulfonamido, C-carboxy, protected C-carboxy, O-carboxy, isocyanato, thiocyanato, isothiocyanato, nitro, silyl, sulfenyl, sulfinyl, sulfonyl, haloalkyl (e.g., mono-, di- and tri-haloalkyl), haloalkoxy (e.g., mono-, di- and tri-haloalkoxy), trihalomethanesulfonyl, trihalomethanesulfonamido, and amino, including mono- and di-substituted amino groups, and the protected derivatives thereof. Wherever a substituent is described as being "optionally substituted" that substitutent may be substituted with one of the above substituents.

As used herein, "aryl" refers to a carbocyclic (all carbon) monocyclic or multicyclic aromatic ring system that has a fully delocalized pi-electron system. Examples of aryl groups include benzene, naphthalene and azulene. An aryl group of this invention may be substituted or unsubstituted. When substituted, hydrogen atoms are replaced by substituent group(s) that is(are) one or more group(s) independently selected from alkyl, alkenyl, alkynyl, cycloalkyl, cycloalkenyl, cycloalkynyl, aryl, heteroaryl, heteroalicyclyl, aralkyl, heteroaralkyl, (heteroalicyclyl)alkyl, hydroxy, protected hydroxy, alkoxy, aryloxy, acyl, ester, mercapto, cyano, halogen, thiocarbonyl, O-carbamyl, N-carbamyl, O-thiocarbamyl, N-thiocarbamyl, C-amido, N-amido, S-sulfonamido, N-sulfonamido, C-carboxy, protected C-carboxy, O-carboxy, isocyanato, thiocyanato, isothiocyanato, nitro, silyl, sulfenyl, sulfinyl, sulfonyl, haloalkyl (e.g., mono-, di- and tri-haloalkyl), haloalkoxy (e.g., mono-, di- and tri-haloalkoxy), trihalomethanesulfonyl, trihalomethanesulfonamido, and amino, including mono- and di-substituted amino groups, and the protected derivatives thereof, unless the substituent groups are otherwise indicated.

A "paramagnetic metal chelate" is a complex wherein a ligand is bound to a paramagnetic metal ion. Examples include 1,4,7,10-Tetraazacyclododecane-1,4,7,10-tetraacetic acid (DOTA)-Gd(III), DOTA-Yttrium-88, DOTA-Indium-111, diethylenetriaminepentaacetic acid (DTPA)-Gd(III), DTPA-yttrium-88, DTPA-Indium-111.

A "polydentate ligand" is a ligand that can bind itself through two or more points of attachment to a metal ion through, for example, coordinate covalent bonds. Examples of polydentate ligands include diethylenetriaminepentaacetic acid (DTPA), tetraazacyclododecane-1,4,7,10-tetraacetic acid (DOTA), (1,2-ethanediyldinitrilo)tetraacetate (EDTA), ethylenediamine, 2,2'-bipyridine (bipy), 1,10-phenanthroline (phen), 1,2-bis(diphenylphosphino)ethane (DPPE), 2,4-pentanedione (acac), and ethanedioate (ox).

A "polydentate ligand precursor with protected oxygen atoms" is a polydentate ligand comprising oxygen atoms, such as the single-bonded oxygen atoms of carboxyl groups, that are protected with suitable protecting groups. Suitable protecting groups include lower alkyls, benzyls, and silyl groups.

A "stabilizing agent" is a substituent that enhances bioavailability and/or prolongs the half-life of a carrier-drug conjugate in vivo by rendering it more resistant to hydrolytic enzymes and less immunogenic. An exemplary stabilizing agent is polyethylene glycol (PEG).

It is understood that, in any compound described herein having one or more chiral centers, if an absolute stereochemistry is not expressly indicated, then each center may independently be of R-configuration or S-configuration or a mixture thereof. Thus, the compounds provided herein may be enatiomerically pure or be stereoisomeric mixtures. In addition it is understood that, in any compound described herein having one or more double bond(s) generating geometrical isomers that can be defined as E or Z each double bond may independently be E or Z a mixture thereof. Likewise, all tautomeric forms are also intended to be included.

The polymer conjugate of the invention comprises at least one recurring unit selected from formula (I), formula (II), and formula (III): 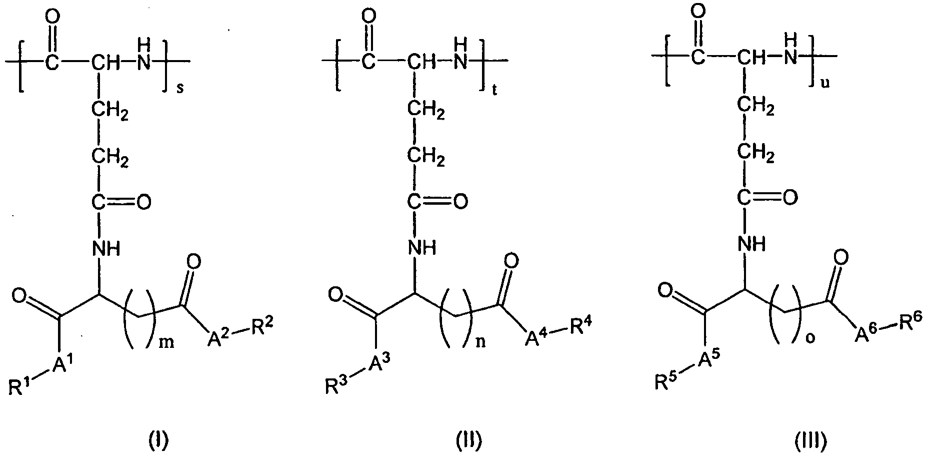 wherein:
A¹, A², A³, A⁴, A⁵ and A⁶ are each independently oxygen or NR⁷, wherein R⁷ is hydrogen or a C₁₋₄ alkyl;
R¹, R², R³, R⁴, R⁵ and R⁶ are each independently selected from the group consisting of a hydrogen, a C₁₋₁₀ alkyl group, a C₆₋₂₀ aryl group, an ammonium group, an alkali metal, a polydentate ligand, a polydentate ligand precursor with protected oxygen atoms, a group that comprises platinum, a group that comprises a drug, a group that comprises a targeting agent, a group that comprises an optical imaging agent, a group that comprises a magnetic resonance imaging agent, and a group that comprises a stabilizing agent;
wherein the polydentate ligand comprises: 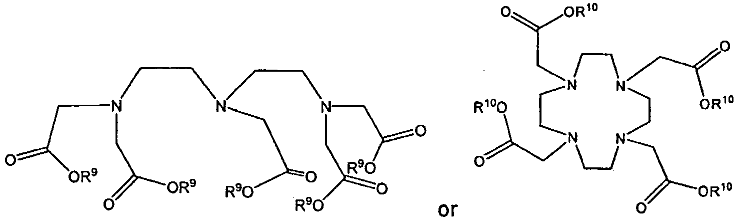 wherein each R⁹ is independently hydrogen, ammonium, or an alkali metal; and
wherein each R¹⁰ is independently hydrogen, ammonium, or an alkali metal;
wherein the polydentate ligand precursor with protected oxygen atoms comprises: 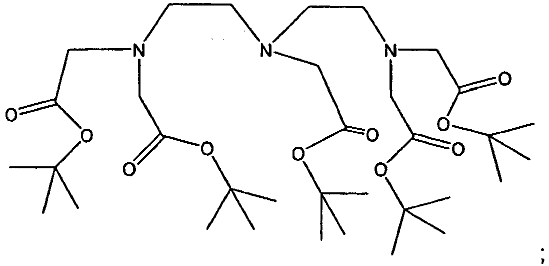 wherein the group that comprises platinum comprises cisplatin, carboplatin, or oxaliplatin, or wherein the recurring unit of formula (I) has the structure: 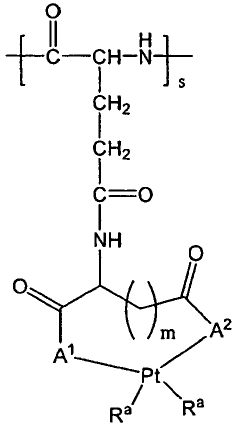 wherein each R^{a} is independently selected from the group consisting of monoalkyl amine, dialkylamine, monoaryl amine, and diaryl amine;
wherein the group that comprises a drug comprises an anticancer drug; wherein the group that comprises the targeting agent is selected from the group consisting of an arginine-glycine-aspartate (RGD) peptide, fibronectin, folate, galactose, an apolipoprotein, insulin, transferrin, a fibroblast growth factor (FGF), an epidermal growth factor (EGF), and an antibody;
wherein the group that comprises an optical imaging agent is selected from the group consisting of an acridine dye, a coumarine dye, a rhodamine dye, a xanthene dye, a cyanine dye, and a pyrene dye;
wherein the group that comprises a magnetic resonance imaging agent
comprises: 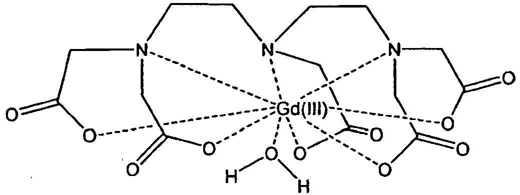 or 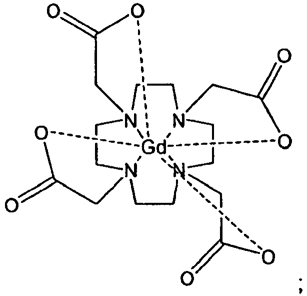 wherein the group that comprises a stabilizing agent comprises polyethylene glycol;
at least one of R¹, R², R³, R⁴, R⁵ and R⁶ is a group that comprises platinum;
m, n, and o are each independently 1 or 2; and
s, t, and u are each independently 0 or ≥ 1, wherein s+t+u is ≥ 1.

In an embodiment, the group that comprises platinum comprises an anticancer drug. In an embodiment, the anticancer drug can be selected from cisplatin (cDDP or cis-diamminedichloroplatinum(II)), carboplatin, oxaliplatin, and combinations thereof

Cisplatin is used to treat a wide variety of conditions. For example, cisplatin can be used in the treatment of testicular, ovarian, and head and neck tumors. Carboplatin is a cisplatin analog in which the chloride ligands are replaced by a 1,1-cyclobutane-dicarboxylic acid chelate. Oxaliplatin is another cisplatin analog wherein the ammonia ligands are replaced with a trans-1R,2R-diaminocyclohexane (1R,2R-DACH) chelate and the chloride ligands are replaced with an oxalic acid chelate. Conjugation of these small molecule platinum complexes to the polymer described herein provides a manner in which the platinum drugs can be targeted to the tumor tissue.

The manner in which the group that comprises platinum may be conjugated to the polymer can vary. In an embodiment, only one of R¹, R², R³, R⁴, R⁵ and R⁶ can be a group that comprises platinum. In another embodiment, the group that comprises platinum can be attached to two or more of A¹, A², A³, A⁴, A⁵ and *A⁶.* For example, the group that comprises platinum may be attached to both A¹ and A², to both A³ and A⁴, or to both A⁵ and A⁶. In an embodiment, s can be 1 or greater and t and u can be 0. In an embodiment, the group that comprises platinum can be attached to A¹ and A². For example, the recurring unit of formula (I) may have the structure: 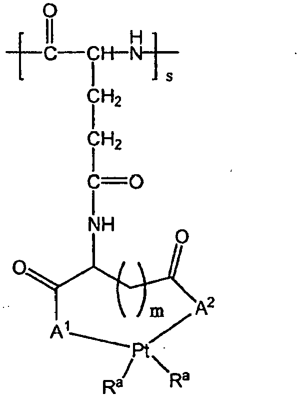 wherein each R^{a} can be independently selected from a monoalkyl amine, a dialkylamine, a monoaryl amine, and a diaryl amine.

In another embodiment, two or more of R¹, R², R³, R⁴, R⁵ and R⁶ can be a group that comprises platinum, wherein the group that comprises platinum is present in more than one recurring unit of the formula (I), (II), and (III). In an embodiment, s + t can be 2 or greater. In an embodiment, the group that comprises platinum can be attached to A² and A³. In another embodiment, the group that comprises platinum can be attached to A² and A⁴. In another embodiment, the group that comprises platinum can be attached to A¹ and A³. In an embodiment where the group that comprises platinum is attached to both the formula (I) and the formula (II), u can be ≥ 1. In an embodiment, at least one of R⁵ and R⁶ can be a group that comprises the drug, wherein the group that comprises platinum and the group that comprises are not the same. In an embodiment, at least one of R⁵ and R⁶ can be selected from the polydentate ligand, the polydentate ligand precursor with protected oxygen atoms, the group that comprises a targeting agent, the group that comprises an optical imaging agent, the group that comprises a magnetic resonance imaging agent, and the group that comprises a stabilizing agent.

In an embodiment, the polymer conjugate can include a cross-linkable unit. In an embodiment, the cross-linkable unit can be capable of undergoing reaction to form a covalent bond with another polymer chain or branch. In an embodiment, the cross-linkable unit can include platinum. In an embodiment, the polymer conjugate can include a cross-linking unit. In an embodiment, the cross-linking unit can form a covalent bond between one polymer chain or branch and another polymer chain or branch. In an embodiment, the cross-linking unit can include platinum.

In an embodiment, s + t can be 2 or greater, u can be 0, and at least one of R¹, R², R³ and R⁴ can be the group that comprises platinum, and at least one of R¹, R², R³ and R⁴ can be the group that comprises a drug, wherein the group that comprises platinum and the group that comprises a drug are not the same.

In an embodiment, s + t can be 2 or greater, u can be 0, and at least one of R¹, R², R³ and R⁴ can be the group that comprises platinum, and at least one of R¹, R², R³ and R⁴ can be selected from the polydentate ligand, the polydentate ligand precursor with protected oxygen atoms, the group that comprises a targeting agent, the group that comprises, an optical imaging agent, the group that comprises a magnetic resonance imaging agent, and the group that comprises a stabilizing agent.

In an embodiment, s + t + u can be 3. In an embodiment, R⁵ and R⁶ can be independently selected from a hydrogen, a C₁₋₁₀ alkyl group, a C₆₋₂₀ aryl group, an ammonium group, and an alkali metal.

A broad variety of other recurring units may be included in the polymer conjugate comprising at least one recurring unit selected from formula (I), formula (II), and formula (III). An embodiment provides a polymer conjugate as described herein, further can include a recurring unit of formula (IV): 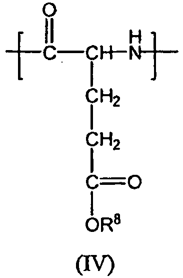 wherein R⁸ can be hydrogen, ammonium, or an alkali metal. When the R⁸ group is hydrogen, then the recurring unit of the formula (IV) is a recurring unit of glutamic acid.

The amount of platinum conjugated to the polymer may vary over a wide range. In an embodiment, the polymer conjugate can include a total amount of the platinum in the range of about 0.5% to about 50% (weight/weight) based on the mass ratio of the platinum to the polymer conjugate (the weight of the platinum is accounted for in the polymer conjugate). In an embodiment, the polymer conjugate can include an amount of the platinum in the range of about 1% to about 40% (weight/weight) based on the mass ratio of the platinum to the polymer conjugate. In an embodiment, the polymer conjugate can include an amount of the platinum in the range of about 1% to about 30% (weight/weight) based on the mass ratio of the platinum to the polymer conjugate. In an embodiment, the polymer conjugate can include an amount of the platinum in the range of about 1% to about 20% (weight/weight) based on the mass ratio of the platinum to the polymer conjugate. In an embodiment, the polymer conjugate can include an amount of the platinum in the range of about 1% to about 10% (weight/weight) based on the mass ratio of the platinum to the polymer conjugate.

In an embodiment, the polymer conjugate can include at least one of the agent, the polydentate ligand, and the polydentate ligand precursor. In an embodiment, the agent can include any one or more selected from an anti-cancer drug, the targeting agent, the optical imaging agent, the magnetic resonance imaging agent, and polyethylene glycol.

One or more of the group that comprises a drug, the group that comprises a targeting agent, the group that comprises an optical imaging agent, the group that comprises a magnetic resonance imaging agent, the groupe that comprises a polydentate ligand, the group that comprises a polydentate ligand precursor, the group that comprises a stabilizing agent and the group that comprises platinum may be conjugated to the polymer in many different ways. In some embodiments, the aforementioned compounds can be directly attached to the polymer, e.g., to a recurring unit of formulae (I), (II) and/or (III). In an embodiment, one or more of the group that comprises a drug, the group that comprises a targeting agent, the group that comprises an optical imaging agent, the group that comprises a magnetic resonance imaging agent, the group that comprises a polydentate ligand, the group that comprises a polydentate ligand precursor, the group that comprises a stabilizing agent and the group that comprises platinum can be directly attached to the polymer. In one embodiment, one or more of the group that comprises a drug, the group that comprises a targeting agent, the group that comprises an optical imaging agent, the group that comprises a magnetic resonance imaging agent, the group that comprises a polydentate ligand, the group that comprises a polydentate ligand precursor, the group that comprises a stabilizing agent and the group that comprises platinum can be directly attached to the polymer through an oxygen, a sulfur, a nitrogen and/or carbon atom of the agent or drug. In other embodiments, one or more of the group that comprises a drug, the group that comprises a targeting agent, the group that comprises an optical imaging agent, the group that comprises a magnetic resonance imaging agent, the group that comprises a polydentate ligand, the group that comprises a polydentate ligand precursor, the group that comprises a stabilizing agent and the group that comprises platinum can further include a linker group. In an embodiment one or more of the group that comprises a drug, the group that comprises a targeting agent, the group that comprises an optical imaging agent, the group that comprises a magnetic resonance imaging agent, the group that comprises a polydentate ligand, the group that magnetic resonance imaging agent, the group comprises a polydentate ligand, the group that comprises a polydentate ligand precursor, the group that comprises a stabilizing agent further can include a linker group. In an embodiment, the group that comprises platinum further can include a linker group. A linker group is a group that attaches, for example, the agent (or the compound that comprises the agent) to the polymer. In an embodiment, one or more of the aforementioned compounds can be attached to the polymer, e.g., to a recurring unit of formulae (I), (II) and/or (III), through a linker group. The linker group may be relatively small. For instance, the linker group may comprise an amine, an amide, an ether, an ester, a hydroxyl group, a carbonyl group, or a thiolether group. Alternatively, the linker group may be relatively large. For instance, the linker group may comprise an alkyl group, an ether group, an aryl group, an aryl(C₁₋₆ alkyl) group (e.g., phenyl-(CH₂)₁₋₄-), a heteroaryl group, or a heteroaryl(C₁₋₆ alkyl) group. In one embodiment, the linker can be -NH(CH₂)₁₋₄-NH-. In another embodiment, the linker can be -(CH₂)₁₋₄-aryl-NH-. The linker group can be attached to one or more of the group that comprises a drug, the group that comprises a targeting agent, the group that comprises an optical imaging agent, the group that comprises a magnetic resonance imaging agent, the group that comprises a polydentate ligand, the group that comprises a polydentate ligand precursor, the group that comprises a stabilizing agent and the group that comprises platinum at any suitable position. For example, the linker group can be attached in place of a hydrogen at a carbon of one of the aforementioned compounds. The linker group can be added to the compounds using methods known to those skilled in the art.

The agent may comprise any type of active compound. In an embodiment, the agent may include the optical imaging agent, which can be one or more selected from an acridine dye, a coumarine dye, a rhodamine dye, a xanthene dye, a cyanine dye, and a pyrene dye. For instance, specific optical imaging agents may include Texas Red, Alexa Fluor® dye, BODIPY® dye, Fluorescein, Oregon Green® dye, and Rhodamine Green^{™} dye, which are commercially available or readily prepared by methods known to those skilled in the art.

In another embodiment, the agent may include the anticancer drug. In an embodiment, the anticancer drug may be selected from a taxane, a camptotheca, and an anthracycline. When the agent comprises a taxane, it is preferable that the taxane is paclitaxel or docetaxel. Paclitaxel may be conjugated to at least one recurring unit selected from formula (I), formula (II), and formula (III) at the oxygen atom via the C2'-carbon of the paclitaxel. Alternatively or in addition, paclitaxel may be conjugated to at least one recurring unit selected from formula (I), formula (II), and formula (III) at the oxygen atom via the C7-carbon of the paclitaxel. When the anticancer drug is a camptotheca, it is preferably camptothecin. In an embodiment when the anticancer drug is anthracycline, it can be doxorubicin.

In another embodiment, the agent may include the targeting agent, which can be one or more selected from an arginine-glycine-aspartate (RGD) peptide, fibronectin, folate, galactose, an apolipoprotein, insulin, transferrin, a fibroblast growth factor (FGF), an epidermal growth factor (EGF), and an antibody. In another preferred embodiment, the targeting agent can interact with a receptor selected from αᵥ,β₃-integrin, folate, asialoglycoprotein, a low-density lipoprotein (LDL), an insulin receptor, a transferrin receptor, a fibroblast growth factor (FGF) receptor, an epidermal growth factor (EGF) receptor, and an antibody receptor. In an embodiment, the arginine-glycine-aspartate (RGD) peptide is cyclic (fKRGD).

In another embodiment, the agent can include the magnetic resonance imaging agent. The magnetic resonance imaging agent includes a paramagnetic metal compound. The magnetic resonance imaging agent comprises a Gd(III) compound selected from:

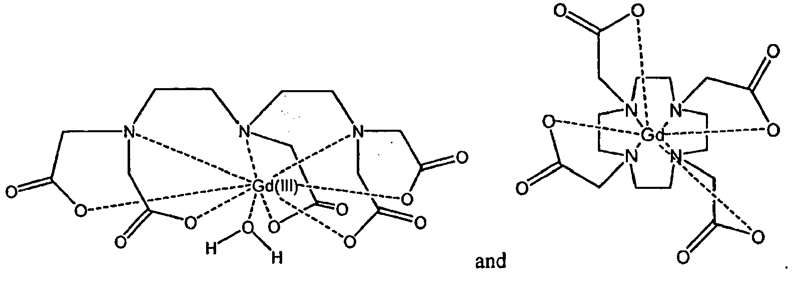

In another embodiment, the agent can include the stabilizing agent, polyethylene glycol.

In another embodiment, the polymer conjugate can include the polydentate ligand. In an embodiment, the polydentate ligand may be capable of reaction with a paramagnetic metal to form a magnetic resonance imaging agent. The polydentate ligand may comprise several carboxylic acid and/or carboxylate groups. The polydentate ligand is selected from: 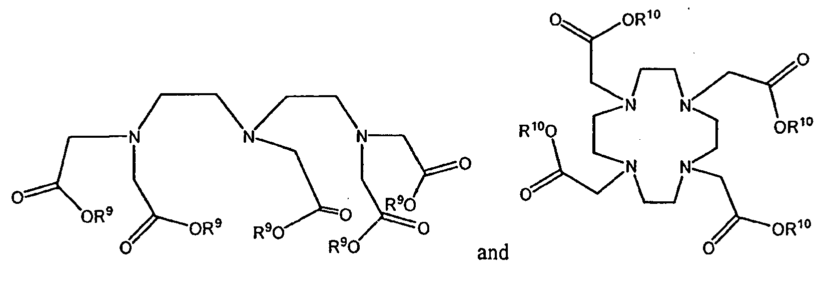 wherein each R⁹ and each R¹⁰ can be independently hydrogen, ammonium, or an alkali metal.

In another embodiment, the polymer conjugate can include the polydentate ligand precursor. In such an embodiment, the oxygen atoms of the polydentate ligand are protected by a suitable protecting group. Suitable protecting groups include lower alkyls, benzyls, and silyl groups. The polydentate ligand precursor having protecting groups is provided as follows:

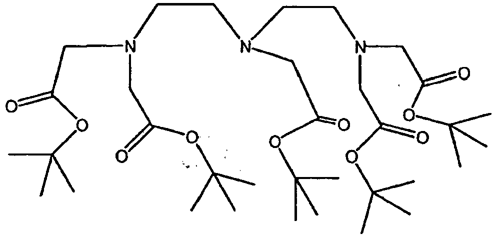

In an embodiment, at least one of m, n, or o can be 1. In an embodiment, at least one of m, n, or o is 2.

In some embodiments, the polymers described herein comprise an alkali metal, for example, lithium (Li), sodium (Na), potassium (K), rubidium (Rb), and cesium (Cs). In an embodiment, the alkali metal may be sodium or potassium. In an embodiment, the alkali metal can be sodium.

The amount of agent(s), such as the drug, the targeting agent, the optical imaging agent, the magnetic resonance imaging agent, and/or the stabilizing agent, present in the polymer can vary over a wide range. Additionally, the amount of the lingand or the ligand precursor present in the polymer can vary over a wide range. In an embodiment, the polymer conjugate can include an amount of the agent(s), the ligand, and/or the ligand precursor in the range of about 0.1% to about 50% (weight/weight) based on the mass ratio of the agent(s), ligand, and/or ligand precursor to the polymer conjugate (the weight of the agent(s), ligand, and/or ligand precursor is accounted for in the polymer conjugate). In an embodiment, the polymer conjugate can include an amount of the agent(s), the ligand, and/or the ligand precursor in the range of about 1% to about 40% (weight/weight) based on the mass ratio of the agent(s), ligand, and/or ligand precursor to the polymer conjugate. In an embodiment, the polymer conjugate can include an amount of the agent(s), the ligand, and/or the ligand precursor in the range of about 1% to about 30% (weight/weight) based on the mass ratio of the agent(s), ligand, and/or ligand precursor to the polymer conjugate. In an embodiment, the polymer conjugate can include an amount of the agent(s), the ligand, and/or the ligand precursor in the range of about 1% to about 20% (weight/weight) based on the mass ratio of the agent(s), ligand, and/or ligand precursor to the polymer conjugate. In an embodiment, the polymer conjugate can include an amount of the agent(s), the ligand, and/or the ligand precursor in the range of about 1% to about 10% (weight/weight) based on the mass ratio of the agent(s), ligand, and/or ligand precursor to the polymer conjugate. In an embodiment, the polymer conjugate can include an amount of the agent(s), the ligand, and/or the ligand precursor in the range of about 5% to about 40% (weight/weight) based on the mass ratio of the agent(s), ligand, and/or ligand precursor to the polymer conjugate. In an embodiment, the polymer conjugate can include an amount of the agent(s), the ligand, and/or the ligand precursor in the range of about 10% to about 30% (weight/weight) based on the mass ratio of the agent(s), ligand, and/or ligand precursor to the polymer conjugate. In an embodiment, the polymer conjugate can include an amount of the agent(s), the ligand, and/or the ligand precursor in the range of about 20% to about 40% (weight/weight) based on the mass ratio of the agent(s), ligand, and/or ligand precursor to the polymer conjugate. In an embodiment, the polymer conjugate can include an amount of the agent(s), the ligand, and/or the ligand precursor in the range of about 30% to about 50% (weight/weight) based on the mass ratio of the agent(s), ligand, and/or ligand precursor to the polymer conjugate.

Polymers comprising at least two of the recurring units of the formula (I), formula (II), and formula (III) are copolymers comprising two or more different recurring units. Further, polymers comprising at least one of the recurring units of the formula (I), formula (II), and formula (III) may be copolymers that comprise other recurring units that are not of the formula (I), formula (II), or formula (III). The number of recurring units of the formula (I), the number of recurring units of the formula (II), and the number of recurring units of the formula (III) can be each independently selected, and may be varied over a broad range. In an embodiment, the number of recurring units of any of the formula (I), formula (II), and formula (III) is in the range of from about 50 to about 5,000, and more preferably from about 100 to about 2,000.

The percentage of recurring units of formula (I) in the polymer conjugate, based on the total number of recurring units, may vary over a wide range. In an embodiment, the polymer conjugate may include up to about 100 mole % of the recurring unit of formula (I), based on the total moles of recurring units in the polymer conjugate. In an embodiment, the polymer conjugate may include about 1 mole % to about 99 mole % of the recurring unit of formula (I), based on the total moles of recurring units in the polymer conjugate. In an embodiment, the polymer conjugate may include about 1 mole % to about 50 mole % of the recurring unit of formula (I) based on the total moles of recurring units of the polymer conjugate. In an embodiment, the polymer conjugate may include about 1 mole % to about 30 mole % of the recurring unit of formula (I) based on the total moles of recurring units of the polymer conjugate. In an embodiment, the polymer conjugate may include about 1 mole % to about 20 mole % of the recurring unit of formula (I) based on the total moles of recurring units of the polymer conjugate. In another embodiment, the polymer conjugate may include about 1 mole % to about 10 mole % of the recurring unit of formula (I) based on the total moles of recurring units of the polymer conjugate.

The percentage of recurring units of formula (II) in the polymer conjugate, based on the total number of recurring units, may also vary over a wide range. In an embodiment, the polymer conjugate may include up to about 100 mole % of the recurring unit of formula (II), based on the total moles of recurring units in the polymer conjugate. In an embodiment, the polymer conjugate may include about 1 mole % to about 99 mole % of the recurring unit of formula (II), based on the total moles of recurring units in the polymer conjugate. In an embodiment, the polymer conjugate may include about 1 mole % to about 50 mole % of the recurring unit of formula (II) based on the total moles of recurring units of the polymer conjugate. In an embodiment, the polymer conjugate may include about 1 mole % to about 30 mole % of the recurring unit of formula (II) based on the total moles of recurring units of the polymer conjugate. In an embodiment, the polymer conjugate may include about 1 mole % to about 20 mole % of the recurring unit of formula (II) based on the total moles of recurring units of the polymer conjugate. In another embodiment, the polymer conjugate may include about 1 mole % to about 10 mole % of the recurring unit of formula (II) based on the total moles of recurring units of the polymer conjugate.

Likewise, the percentage of recurring units of formula (III) in the polymer conjugate, based on the total number of recurring units, may vary over a wide range. In an embodiment, the polymer conjugate may include up to about 100 mole % of the recurring unit of formula (III), based on the total moles of recurring units in the polymer conjugate. In an embodiment, the polymer conjugate may include about 1 mole % to about 99 mole % of the recurring unit of formula (III), based on the total moles of recurring units in the polymer conjugate. In an embodiment, the polymer conjugate may include about 1 mole % to about 50 mole % of the recurring unit of formula (III) based on the total moles of recurring units of the polymer conjugate. In an embodiment, the polymer conjugate may include about 1 mole % to about 30 mole % of the recurring unit of formula (III) based on the total moles of recurring units of the polymer conjugate. In an embodiment, the polymer conjugate may include about 1 mole % to about 20 mole % of the recurring unit of formula (III) based on the total moles of recurring units of the polymer conjugate. In another embodiment, the polymer conjugate may include about 1 mole % to about 10 mole % of the recurring unit of formula (III) based on the total moles of recurring units of the polymer conjugate.

In some embodiment, the polymer may include a recurring unit of the formula (IV). The percentage of recurring units of formula (IV) in the polymer conjugate, based on the total number of recurring units, may vary over a wide range. In an embodiment, the polymer conjugate may include up to about 99 mole % of the recurring unit of formula (IV), based on the total moles of recurring units in the polymer conjugate. In an embodiment, the polymer conjugate may include about 1 mole % to about 99 mole % of the recurring unit of formula (IV), based on the total moles of recurring units in the polymer conjugate. In an embodiment, the polymer conjugate may include about 1 mole % to about 50 mole % of the recurring unit of formula (IV) based on the total moles of recurring units of the polymer conjugate. In an embodiment, the polymer conjugate may include about 1 mole % to about 30 mole % of the recurring unit of formula (IV) based on the total moles of recurring units of the polymer conjugate. In an embodiment, the polymer conjugate may include about 1 mole % to about 20 mole % of the recurring unit of formula (IV) based on the total moles of recurring units of the polymer conjugate. In another embodiment, the polymer conjugate may include about 1 mole % to about 10 mole % of the recurring unit of formula (IV) based on the total moles of recurring units of the polymer conjugate.

In an embodiment, the polymer conjugates described herein (e.g., a polymer conjugate comprising units of formula (I), formula (II), and/or formula (III)) can further include an agent that can activate PPARγ. Suitable agents include rosizitaglone and pioglitazone. The agent that can activate PPARγ can be included in polymer conjugates described herein in various ways. For example, the agent that can activate PPARγ can be convalently bonded to the polymer conjugate (e.g., a polymer conjugate comprising units of formula (I), formula (II), and/or formula (III)) directly or through a linker group such as those described herein. Alternatively, the agent that can activate PPARγ can be can be non-covalently encapsulated or partially encapsulated within a polymer matrix of the polymer conjugate described herein.

In an embodiment, the platinum compound can be non-covalently encapsulated or partially encapsulated within a polymer matrix of the polymer conjugate described herein. For example, the polymer conjugates described herein may be present in various forms, including in the form of particles, flakes, rods, fibers, films, foams, suspensions (in liquid or gas), a gel, a solid, or a liquid. The size and shape of these various forms is not limited. Free and non-conjugated platinum compounds, such as cisplatin, carboplatin, and oxaliplatin, may be mixed with the polymer conjugate described herein as it forms a matrix and be non-covalently encapsulated or partially encapsulated therein.

In an embodiment, the amount of the agent(s) and the percentage amounts of the recurring units of the formula (I), formula (II), and/or formula (III) may be selected to advantageously control the solubility of the resulting polymer conjugate. For example, in preferred embodiments, the amount of the agent(s) and the percentage amounts of the recurring units of the formula (I), formula (II), and/or formula (III) can be selected so that the polymer conjugate is soluble (or insoluble) at a particular pH and/or pH range of interest. In some embodiments, the molecular weight of the polymer can be also selected to control solubility. Those skilled in the art, informed by the guidance provided herein, can use routine experimentation to identify suitable amounts of the agent(s) and percentage amounts of the recurring units of the formula (I), formula (II), and/or formula (III) that result in a polymer conjugate with desired solubility characteristics. Such control over solubility may be advantageous, depending on the application. For example, embodiments of the polymer conjugates provided herein may be used to provide improved delivery of otherwise poorly soluble anticancer drugs to selected tissues, preferably reducing undesired side effects, and/or may reduce the frequency at which a subject needs to take the anticancer drug.

The amount of the agent(s) and the percentage amounts of the recurring units of the formula (I), formula (II), and/or formula (III) can be preferably selected to provide a polymer conjugate solubility that is greater than that of a comparable polyglutamic acid conjugate that comprises substantially the same amount of the same agent(s). In an embodiment, the polymer conjugate solubility is greater than that of a comparable polyglutamic acid conjugate. Solubility is measured by forming a polymer conjugate solution comprising at least 5 mg/mL of the polymer conjugate in 0.9 wt. % aqueous NaCl at about 22°C, and determining the optical clarity. Optical clarity may be determined turbidimetrically, e.g., by visual observation or by appropriate instrumental methods known to those skilled in the art. Comparison of the resulting solubility to a similarly formed polyglutamic acid conjugate solution shows improved solubility as evidenced by greater optical clarity over a broader range of pH values. Thus, a polymer conjugate solubility is greater than that of a comparable polyglutamic acid conjugate that comprises substantially the same amount of the agent when a tested polymer conjugate solution, comprising at least 5 mg/mL of the polymer conjugate in 0.9 wt. % aqueous NaCl at about 22°C, has greater optical clarity over a broader pH range than that of a comparable tested polyglutamic acid conjugate solution. Those skilled in the art will understand that a "comparable" polyglutamic acid conjugate is a control material in which the polymeric portion of the conjugate has a molecular weight that is approximately the same as that of the subject polymer conjugate (comprising a recurring unit of the formula (I), formula (II), and/or formula (III)) to which it is being compared.

The polymer conjugate can contain one or more chiral carbon atoms. The chiral carbon (which may be indicated by an asterisk *) can have the *rectus* (right handed) or the *sinister* (left handed) configuration, and thus the recurring unit may be racemic, enantiomeric or enantiomerically enriched. The symbols "n" and "*" (designating a chiral carbon), as used elsewhere herein, have the same meaning as specified above, unless otherwise stated.

Polymers comprising at least one recurring unit selected from formula (I), formula (II), and formula (III) may be prepared in various ways. In an embodiment, a polymeric reactant can be dissolved or partially dissolved in a solvent to form a dissolved or partially dissolved polymeric reactant. The dissolved or partially dissolved polymeric reactant can be then reacted with a second reactant to form an intermediate product or, in some embodiments, a polymer comprising at least one recurring unit selected from formula (I), formula (II), and formula (III). In an embodiment, the second reactant can include the group that comprises platinum. In an embodiment, the group that comprises platinum can be a group comprising cisplatin. In an embodiment, the group that comprises platinum can be a group comprising carboplatin. In an embodiment, the group that comprises platinum can be a group comprising oxaliplatin. In an embodiment, various combinations of a group that comprises cisplatin, a group that comprises carboplatin, and a group that comprises oxaliplatin may be conjugated to the polymers described herein.

The polymeric reactant may include any suitable material capable of forming a polymer comprising at least one recurring unit selected from formula (I), formula (II), and formula (III). In an embodiment, the polymeric reactant may include a recurring unit of the formula (V): 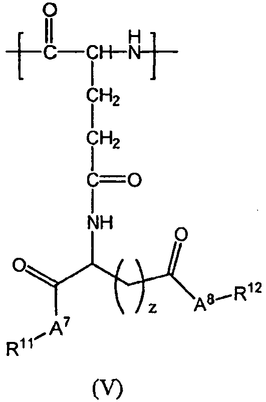 wherein z can be independently 1 or 2; A⁷ and A⁸ are oxygen; and R¹¹ and R¹² can be each independently selected from hydrogen, ammonium, and an alkali metal.

In an embodiment, the dissolved or partially dissolved polymeric reactant can be reacted with a second reactant, wherein the second reactant comprises the group comprising platinum. In an embodiment, the second reactant can include a substituent selected from a hydroxy and an amine.

In an embodiment, the group that comprises platinum can be an anti-cancer drug. In an embodiment, the group that comprises platinum can be cisplatin. In an embodiment, the group that comprises platinum can be carboplatin. In an embodiment, the group that comprises platinum can be oxaliplatin.

Mixtures of free (non-conjugated) platinum compounds, such as cisplatin, carboplatin, and oxaliplatin, with the polymer conjugates described herein may be formed in various ways, e.g., to form a matrix in which some or all of the platinum compound is non-covalently encapsulated or partially encapsulated therein. Such a mixture may contain, for example, both conjugated and non-conjugated drug. The polymer conjugate may be dissolved or partially dissolved in a variety of solvents to prepare it for mixture with the group that comprises platinum. In an embodiment, the solvent can include a hydrophilic solvent, such as a polar solvent. Suitable polar solvents include protic solvents such as water, methanol, ethanol, propanol, isopropanol, butanol, formic acid, and acetic acid. Other suitable polar solvents include aprotic solvents, such as acetone, acetonitrile, dimethylformamide, dimethyl sulfoxide, tetrahydrofuran, and 1,4-dioxane. In an embodiment, the solvent can be an aqueous solvent, for example, water.

Dissolving or partial dissolving the polymer conjugate in a solvent may be further aided by the use of conventional mechanical techniques. For instance, the polymer conjugate may be shaken or stirred in the solvent to induce dissolving or partial dissolving. In an embodiment, the polymer and solvent are sonicated. Sonication is the act of applying sound energy, for example, ultrasound energy, to agitate the particles in a sample. Sonication may take place using, for example, an ultrasonic bath or an ultrasonic probe. The degree to which the polymer is dissolved may be controlled by varying the intensity and duration of the mechanical shaking or stirring or the sonication conditions. Shaking, stirring, or sonicating may take place over any duration of time. For example, the mixture may be sonicated for a period of time ranging between several seconds to several hours. In an embodiment, the polymer conjugate can be sonicated in the solvent for a period of time ranging between about 1 minute and about 10 minutes. In an embodiment, the polymer conjugate can be sonicated in the solvent for about 5 minutes.

In an embodiment, the group that comprises platinum can be added to the polymer conjugate solution. The group that comprises platinum may or may not be dissolved or partially dissolved in solvent(s) before it is mixed with the polymer conjugate. If the group that comprises platinum is dissolved or partially dissolved in a solvent, the solvent may inlcude a hydrophilic solvent, such as a polar solvent. Suitable polar solvents include protic solvents such as water, methanol, ethanol, propanol, isopropanol, butanol, formic acid, acetic acid, and acetone. Other suitable polar solvents include aprotic solvents, such as acetone, acetonitrile, dimethylformamide, dimethyl sulfoxide, tetrahydrofuran, and 1,4-dioxane. In an embodiment, the group that comprises platinum can be dissolved or partially dissolved in an alcohol. In an embodiment, the group that comprises platinum can be dissolved or partially dissolved in ethanol.

After the group that comprises platinum is added to the polymer conjugate solution, for example, by using a pipette, additional mixing may be performed. For instance, the polymer conjugate and group that comprises platinum solution may be shaken or stirred. In an embodiment, the polymer conjugate and group that comprises platinum solution can be sonicated. Shaking, stirring, or sonicating may take place over any duration of time. For instance, the mixture may be sonicated for a period of time ranging between several seconds to several hours. In an embodiment, the polymer conjugate and group that comprises platinum solution can be sonicated for a period of time ranging between about 1 minute and about 10 minutes. In an embodiment, the polymer conjugate and group that comprises platinum solution can be sonicated for about 5 minutes.

In an embodiment, the polymer conjugate and group that comprises platinum are mixed together before either is dissolved in a solvent. In an embodiment, a solvent or mixture of solvents may be added to the mixture of the polymer conjugate and group that comprises platinum. After the solvent or mixture of solvents is added to the polymer conjugate and group that comprises platinum, one of or both of the polymer conjugate and group that comprises platinum may be dissolved or partially dissolved. The solvent or mixture of solvents may include one or more of water, methanol, ethanol, propanol, isopropanol, butanol, formic acid, acetic acid, acetone, acetonitrile, dimethylformamide, dimethyl sulfoxide, tetrahydrofuran, and 1,4-dioxane. In an embodiment, the mixture of solvents can include an alcohol and water. In an embodiment, the mixture of solvents can include ethanol and water.

Optionally, the polymer conjugate comprising platinum may then be isolated and/or purified. Suitable methods known to those skilled in the art can be used to isolate and/or purify the polymer conjugate comprising platinum. The composition may then be dried by any suitable method known to those skilled in the art. For example, in one embodiment, the polymer conjugate comprising platinum can be freeze-dried. The conditions of freeze-drying the composition may vary. In an embodiment, the mixture can be freeze-dried at a temperature ranging between about -30°C to about -10°C. In an embodiment, the mixture can be freeze-dried at a temperature of about -20°C. Once the polymer conjugate comprising platinum has been optionally isolated and dried, it may then be stored in appropriate conditions. For example, the composition may be stored in at a temperature suitable for freeze-drying, as set forth above.

In an embodiment, the dissolved or partially dissolved polymeric reactant can be reacted with a third reactant. The reaction with a third reactant may take place before, at about the same time, or after the dissolved or partially dissolved polymeric reactant is reacted with the second reactant. The third reactant may include a variety of compounds. In an embodiment, the third reactant can include the group that comprises an agent. The agent may be any active compound. For example, the agent may be selected from the group consisting of an anti-cancer drug, the targeting agent, the optical imaging agent, the magnetic resonance imaging agent, the stabilizing agent, and an agent that activates PPARγ (e.g, rosizitaglone and pioglitazone). In some embodiments, the third reactant can include the polydentate ligand or the polydentate ligand precursor with protected oxygen atoms. In an embodiment, the third reactant can include an agent that activates PPARγ, such as rosizitaglone and pioglitazone. In an embodiment, the third reactant may include a substituent. The substituent may be selected from hydroxy and an amine.

In an embodiment, the dissolved or partially dissolved polymeric reactant can be reacted with a fourth reactant. The reaction with a third reactant may take place before, at about the same time, or after the dissolved or partially dissolved polymeric reactant is reacted with the fourth reactant. The fourth reactant may include a variety of compounds. In an embodiment, the third fourth can include the group that comprises an agent. The agent may be any active compound. For example, the agent may be selected from the group consisting of an anti-cancer drug, the targeting agent, the optical imaging agent, the magnetic resonance imaging agent, the stabilizing agent, and an agent that activates PPAR (e.g, rosizitaglone and pioglitazone). In some embodiments, the fourth reactant can include the polydentate ligand or the polydentate ligand precursor with protected oxygen atoms. In an embodiment, the fourth reactant may include a substituent. In an embodiment, the fourth reactant can include an agent that activates PPARγ, such as rosizitaglone and pioglitazone. The substituent may be selected from hydroxy and an amine.

In an embodiment, the anticancer drug can be selected from a taxane, camptotheca, and anthracycline. In an embodiment, the anticancer drug may include taxane, and the taxane may be selected from paclitaxel and docetaxel. Paclitaxel may be conjugated to the polymer in a number of ways. In an embodiment, paclitaxel can be conjugated to at least one recurring unit selected from formula (I), formula (II), and formula (III) at the oxygen atom attached to the C2'-carbon. In another embodiment, paclitaxel can be conjugated to at least one recurring unit selected from formula (I), formula (II), and formula (III) at the oxygen atom attached to the C7-carbon. In an embodiment, the anticancer drug can be a camptotheca, for example, camptothecin. In an embodiment, the anticancer drug can be anthracycline, such as doxorubicin. In an embodiment, the group that comprises the drug and the group that comprises platinum are not the same.

The targeting agent is selected from an arginine-glycine-aspartate (RGD) peptide, fibronectin, folate, galactose, an apolipoprotein, insulin, transferrin, a fibroblast growth factor (FGF), an epidermal growth factor (EGF), and an antibody. In an embodiment, the targeting agent can interact with a receptor selected from αᵥ,β₃-integrin, folate, asialoglycoprotein, a low-density lipoprotein (LDL), an insulin receptor, a transferrin receptor, a fibroblast growth factor (FGF) receptor, an epidermal growth factor (EGF) receptor, and an antibody receptor. In some embodiments, the arginine-glycine-aspartate (RGD) peptide can be cyclic (fKRGD).

The optical imaging agent is selected from an acridine dye, a coumarine dye, a rhodamine dye, a xanthene dye, a cyanine dye, and a pyrene dye. In an embodiment, the stabilizing agent can be polyethylene glycol.

In an embodiment, the compound that comprises the agent can include the magnetic resonance imaging agent. The magnetic resonance imaging includes a paramagnetic metal compound. The magnetic resonance imaging agent includes a Gd(III) compound selected from the following:

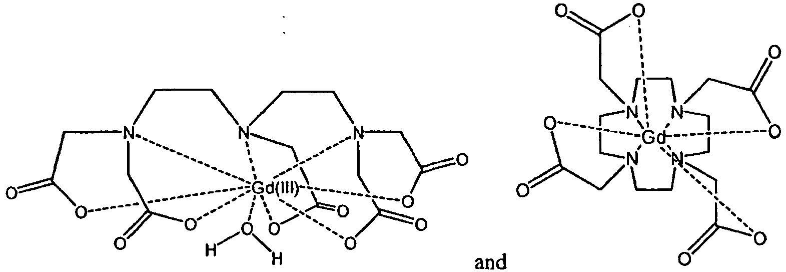

In an embodiment, the polydentate ligand may be conjugated to the polymer. In an embodiment, the polydentate ligand may be capable of reaction with a paramagnetic metal to form a magnetic resonance imaging agent. For example, the polydentate ligand may comprise several carboxylic acid and/or carboxylate groups. The polydentate ligands of the following structures may be conjugated to the polymer: 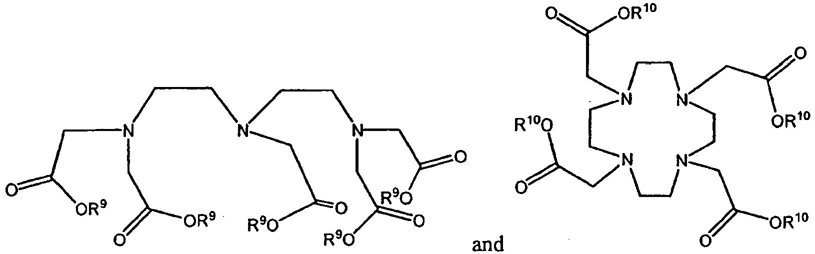 wherein each R⁹ and each R¹⁰ are independently hydrogen, ammonium, or an alkali metal.

In another embodiment, the polydentate ligand precursor having protecting groups may be conjugated to the polymer. Such a precursor has its oxygen atoms protected by a suitable protecting group(s). Suitable protecting groups include lower alkyls, benzyls, and silyl groups. The polydentate ligand precursor having protecting groups is provided as follows:

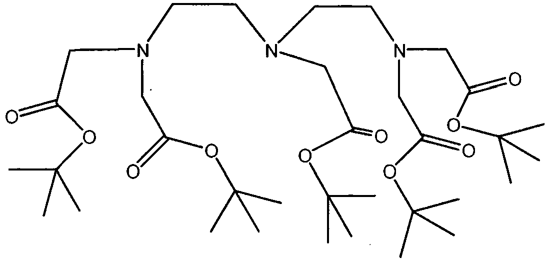

In some embodiments, the dissolved or partially dissolved polymer reactant can be reacted with at least a portion of the second reactant before reacting with the third reactant. In an embodiment, the intermediate compound that forms after the addition of at least a portion of the second reactant can be isolated before adding the third reactant. In another embodiment, the third reactant can be added without isolating the intermediate compound that forms after the addition of the second reactant. In other embodiments, the dissolved or partially dissolved polymer reactant can be reacted with at least a portion of the second reactant at about the same time as reacting with the third reactant. In an embodiment, the dissolved or partially dissolved polymer reactant can be reacted with at least a portion of the second reactant after reacting with the third reactant. In an embodiment, the intermediate compound that forms after the addition of at least a portion of the third reactant can be isolated before adding the second reactant.

In an embodiment, a method of making the polymer conjugate can include reacting the dissolved or partially dissolved polymeric reactant with the second reactant and/or third reactant in the presence of a coupling agent. Any suitable coupling agent may be used. In an embodiment, the coupling agent is selected from 1-ethyl-3-(3-dimethylaminopropyl)-carbodiimide (EDC), 1,3-dicyclohexyl carbodiimide (DCC), 1,1'-carbonyl-diimidazole (CDI), N,N'-disuccinimidyl carbonate (DSC), N-[(dimethylamino)-1H-1,2,3-triazolo-[4,5-b]pyridine-1-yl-methylene]-N-methylmethanaminium hexafluorophosphate N-oxide (HATU), 2-[(1H-benzotriazol-1-yl)-1,1,3,3-tetramethylaminium hexafluorophosphate (HBTU), 2-[(6-chloro-1H-benzotriazol-1-yl)-1,1,3,3-tetramethylaminium hexafluorophosphate (HCTU), benzotriazole-1-yl-oxy-tris-pyrrolidino-phosphonium hexafluorophosphate (PyBOP®), bromo-tris- pyrrolidino-phosphonium hexafluorophosphate (PyBroP®), 2-[(1H-benzotriazol-1-yl)-1,1,3,3-tetramethylaminium tetrafluoroborate (TBTU), and benzotriazol-1-yl-oxy-tris-(dimethylamino)phosphonium hexafluorophosphate (BOP).

Any suitable solvent that allows the reaction to take place may be used. In an embodiment, the solvent may be a polar aprotic solvent. For instance, the solvent may be selected from N,N-dimethylformamide (DMF), dimethyl sulfoxide (DMSO), N-methyl-2-pyridone (NMP), and N,N-dimethylacetamide (DMAc).

In another embodiment, the reaction may further include reacting the dissolved or partially dissolved polymeric reactant in the presence of a catalyst. Any catalyst that promotes the reaction may be used. In an embodiment, the catalyst may comprise 4-dimethylaminopyridine (DMAP).

In an embodiment, a polymer comprising at least one recurring unit selected from formula (I), formula (II), and formula (III) can be produced starting with polyglutamic acid and an amino acid such as asparatic and/or glutamic acid. Alternatively, in another embodiment, the polymer may be created by first converting the starting polyglutamic acid material into its salt form. The salt form of polyglutamic can be obtained by reacting polyglutamic acid with a suitable base, e.g., sodium bicarbonate. An amino acid moiety can be attached to the pendant carboxylic acid group of the polyglumatic acid. The weight average molecular weight of the polyglutamic acid may vary over a broad range, but is preferably from about 10,000 to about 500,000 daltons, and more preferably from about 25,000 to about 300,000 daltons. Such a reaction may be used to create poly-(y-L-aspartyl-glutamine) or poly-(γ-L-glutamyl-glutamine).

In an embodiment, the amino acid is protected by a protecting group before attachment to the polyglutamic acid. One example of a protected amino acid moiety suitable for this reaction is L-aspartic acid di-t-butyl ester hydrochloride, shown below:

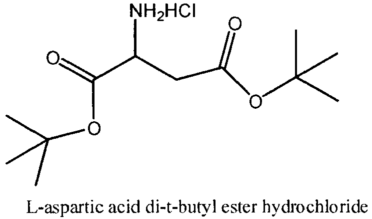

Reaction of the polyglutamic acid with the amino acid may take place in the presence of any suitable solvent. In an embodiment, the solvent can be an aprotic solvent. In a preferred embodiment, the solvent can be N,N'-dimethylformamide.

In an embodiment, a coupling agent such as EDC, DCC, CDI, DSC, HATU, HBTU, HCTU, PyBOP®, PyBroP®, TBTU, and BOP can be used. In other embodiments, polyglutamic acid and an amino acid can be reacted using a catalyst (e.g., DMAP).

After completion of the reaction, if the oxygen atoms of the amino acid are protected, the protecting groups can be removed using known methods such as using a suitable acid (e.g., trifluoroacetic acid). If desired, the salt form of the polymer obtained from reacting polyglutamic acid with the amino acid can be formed by treating the acid form of the polymer with a suitable base solution, e.g., sodium bicarbonate solution.

The polymer may be recovered and/or purified by methods known to those skilled in the art. For example, the solvent may be removed by suitable methods, for instance, rotary evaporation. Additionally, the reaction mixture may be filtered into an acidic water solution to induce precipitation. The resultant precipitate can then be filtered, and washed with water.

In some embodiments, a polymer comprising at least one recurring unit selected from formula (I), formula (II), and formula (III) can also include a recurring unit of formula (IV) as set forth above. One method for forming a polymer comprising (1) at least one recurring unit selected from formula (I), formula (II), and formula (III) and (2) a recurring unit of the formula (IV) is by starting with polyglutamic acid and reacting it with an amino acid such as asparatic and/or glutamic acid, in an amount that is less than 1.0 equivalents of the amino acid based on polyglutamic acid. For example, in one embodiment, 0.7 equivalents of an amino acid based on the polyglutamic acid can be reacted with polyglutamic acid, so that about 70% of the recurring units of the resulting polymer comprise the amino acid. As discussed above, the oxygen atoms of the amino acid can be protected using a suitable protecting group. In an embodiment, the amino acid may be L-aspartic acid or L-glutamic acid. In another embodiment, the oxygen atoms of the amino acid can be protected with t-butyl groups. If the oxygen atoms of the amino acid are protected, the protecting groups can be removed using known methods such as a suitable acid (e.g., trifluoroacetic acid).

Conjugation of the group comprising platinum, the agent, the polydentate ligand, and/or the polydentate ligand precursor with protected oxygen atoms to the polymer acid or its salt form may be carried out in various ways, e.g., by covalently bonding the group comprising platinum, the agent, the polydentate ligand, and/or the polydentate ligand precursor with protected oxygen atoms to various polymers. One method for conjugating the aforementioned groups to the polymer obtained from polyglutamic acid and/or salt is by using heat (e.g, heat from using a microwave method). Alternatively, conjugation may take place at room temperature. Appropriate solvents, coupling agents, catalysts, and/or buffers as generally known to those skilled in the art and/or as described herein may be used to form the polymer conjugate. As with polyglutamic acid, both the salt or acid form of the polymer obtained from polyglutamic acid and/or salt and an amino acid can be used as starting material for forming the polymer conjugate.

Suitable agents that can be conjugated to a polymer comprising at least one recurring unit selected from formula (I), formula (II), and formula (III) (e.g., the polymer obtained from polyglutamic acid and/or salt and an amino acid) include the above drugs, optical agents, targeting agents, magnetic resonance imaging agents (e.g, paramagnetic metal compounds), stabilizing agents, polydentate ligands, and polydentate ligand precursors with protected oxygen atoms.

In one embodiment, a polymer described herein (e.g., the polymer obtained from polyglutamic acid and/or salt and an amino acid) can be conjugated to the optical imaging agent described herein. In an embodiment, the optical agent can be Texas Red-NH₂.

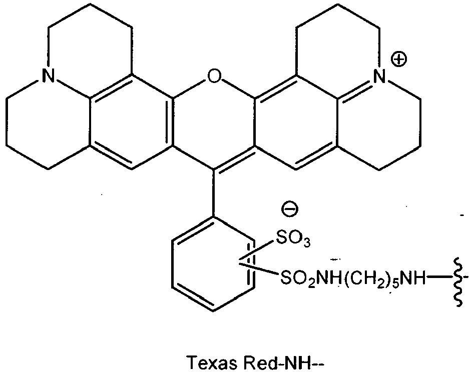

In one particular embodiment, a polymer comprising at least one recurring unit selected from formula (I), formula (II), and formula (III) may be reacted with DCC, Texas Red-NH₂ dye, pyridine, and 4-dimethylaminopyridine. The mixture can be heated using a microwave method. In an embodiment, the reaction can be heated up to a temperature in the range of about 100° -150°C. In another embodiment, the time the materials can be heated ranges from 5 to 40 minutes. If desired, the reaction mixture can be cooled to room temperature. Suitable methods known to those skilled in the art can be used to isolate and/or purify the polymer conjugate. For instance, reaction mixture can be filtered into an acidic water solution. Any precipitate that forms can then be filtered and washed with water. Optionally, the precipitate can be purified by any suitable method. For example, the precipitate can be transferred into acetone and dissolved, and the resulting solution can be filtered again into a sodium bicarbonate solution. If desired, the resulting reaction solution can be dialyzed in water using a cellulose membrane and the polymer can be lyophilized and isolated.

In an embodiment, a polymer comprising at least one recurring unit selected from formula (I), formula (II), and formula (III) (e.g, the polymer obtained from polyglutamic acid and/or salt and an amino acid) can be conjugated to another drug (e.g., another anticancer drug). In an embodiment, the anticancer drug can be a taxane, camptotheca, and/or anthracycline. In an embodiment, the anticancer drug can be a taxane such as paclitaxel or docetaxel. In other embodiments, the anticancer drug conjugated to the polymer can be a camptotheca such as camptothecin. In some embodiments, the anticancer drug conjugated to the polymer can be an anthracycline such as doxorubicin. In other embodiments, the anticancer drug conjugated to the polymer can be paclitaxel. In an embodiment, paclitaxel may be joined to the polymer at the C2'-oxygen atom. In another embodiment, the paclitaxel may be joined to the polymer at the C7-oxygen atom. In another embodiment, the polymer comprises paclitaxel that is coupled to the polymer only by the C2'-oxygen atom. In still another embodiment, the polymer comprises paclitaxel that is coupled to the polymer only by the C7-oxygen atom. In yet another embodiment, the polymer comprises both C2'-conjugated paclitaxel groups and C7-conjugated paclitaxel groups.

In an embodiment, the anti-cancer drug can be conjugated to a polymer described herein (e.g., the polymer obtained from polyglutamic acid and/or salt and an amino acid) using the methods described above with respect to Texas-Red.

In an embodiment, paclitaxel, preferably in the presence of a coupling agent (e.g, EDC and/or DCC) and a catalyst (e.g, DMAP), can be reacted with a polymer comprising at least one recurring unit selected from formula (I), formula (II), and formula (III) (e.g., the polymer obtained from polyglutamic acid and/or salt and an amino acid) in a solvent (e.g, an aprotic solvent such as DMF). Additional agents, such as pyridine or hydroxybenzotriazole may be used. In one embodiment, the reaction may take place over the period of 0.5-2 days. Suitable methods known to those skilled in the art can be used to isolate and/or purify the polymer conjugate. For example, the reaction mixture can be poured into an acidic solution to form a precipitate. Any precipitate that forms can then be filtered and washed with water. Optionally, the precipitate can be purified by any suitable method. For example, the precipitate can be transferred into acetone and dissolved, and the resulting solution can be filtered again into a sodium bicarbonate solution. If desired, the resulting reaction solution can be dialyzed in water using a cellulose membrane and the polymer can be lyophilized and isolated. The content of paclitaxel in the resulting polymer may be determined by UV spectrometry.

Alternatively, the compound comprising the agent can be reacted with an amino acid such as glutamic and/or aspartic acid in which the compound comprising the agent is coupled (e.g., covalently bonded) to the amino acid. The amino acid-agent compound can then be reacted with polyglutamic acid or its salt to form the polymer conjugate. In one embodiment, paclitaxel can be reacted with glutamic acid to form a compound in which the paclitaxel is covalently bonded to the pendant carboxylic acid group of the glutamic acid. The glutamic acid-paclitaxel compound can then be reacted with polyglutamic acid or its salt to form the polymer conjugate. In one embodiment, paclitaxel can be reacted with aspartic acid to form a compound in which the paclitaxel is covalently bonded to the pendant carboxylic acid group of the aspartic acid. The aspartic acid-paclitaxel compound can then be reacted with polyglutamic acid or its salt to form the polymer conjugate. If desired, the paclitaxel coupled to the amino acid by the C2'-oxygen can be separated from the paclitaxel coupled to the amino acid by the C7-oxygen using known separation methods (e.g, HPLC).

After formation of the polymer conjugate, any free amount of agent not covalently bonded to the polymer may also be measured. For example, thin layer chromatography (TLC) may be used to confirm the substantial absence of free paclitaxel remaining in the compositions of polymers conjugated to paclitaxel. Other methods known to those skilled in the art may be used to confirm the substantial absence of free platinum.

In one embodiment, a polymer comprising at least one recurring unit selected from formula (I), formula (II), and formula (III) (e.g., the polymer obtained from polyglutamic acid and/or salt and an amino acid) can be conjugated to the polydentate ligand. Polydentate ligands include diethylenetriaminepentacetic acid (DTPA), tetraazacyclododecane-1,4,7,10-tetraacetic acid (DOTA), (1,2-ethanediyldinitrilo)tetraacetate (EDTA), ethylenediamine, 2,2'-bipyridine (bipy), 1,10-phenanthroline (phen), 1,2-bis(diphenylphosphino)ethane (DPPE), 2,4-pentanedione (acac), and ethanedioate (ox). Appropriate solvents, coupling agents, catalysts, and/or buffers as generally known to those skilled in the art and/or described herein may be used to form the polymer conjugate. In another embodiment, the polymer obtained from polyglutamic acid and/or salt and an amino acid can be conjugated to the polydentate ligand precursor with protected oxygen atoms. As with polyglutamic acid, both the salt or acid form of the polymer obtained from polyglutamic acid and/or salt and an amino acid can be used as starting material for forming the polymer conjugate.

In an embodiment, the polydentate ligand can be DTPA. In another embodiment, the polydentate ligand can be DOTA. In one embodiment, the polydentate ligand such as DTPA (with or without protected oxygen atoms), preferably in the presence of a coupling agent (e.g, DCC) and a catalyst (e.g, DMAP), can be reacted with the polymer obtained from polyglutamic acid and/or salt and an amino acid in a solvent (e.g, an aprotic solvent such as DMF). If protecting groups are present, removal can achieved using suitable methods. For example, the polymer conjugate with the polydentate ligand precursor with protected oxygen atoms such as DTPA with oxygen atoms protected by t-butyl groups can be treated with acid such as trifluoroacetic acid. After removal of the protecting groups, the acid can be removed by rotary evaporation. In one embodiment, DTPA can be treated with a suitable base to remove the hydrogen atoms on the carboxylic acid -OH groups. In some embodiments, the base can be sodium bicarbonate.

In one embodiment, a polymer comprising at least one recurring unit selected from formula (I), formula (II), and formula (III) (e.g., the polymer obtained from polyglutamic acid and/or salt and an amino acid) can be conjugated to the targeting agent. Selected from arginine-glycine-aspartate (RGD) peptides, fibronectin, folate, galactose, apolipoprotein, insulin, transferrin, fibroblast growth factors (FGF), epidermal growth factors (EGF), and antibodies. Targeting agents can be chosen such that they interact with particular receptors. For example, the targeting agent can be chosen so that it interacts with one or more of the following receptors: αᵥ,β₃-integrin, folate, asialoglycoprotein, a low-density lipoprotein (LDL), an insulin receptor, a transferrin receptor, a fibroblast growth factor (FGF) receptor, an epidermal growth factor (EGF) receptor, and an antibody receptor. In one embodiment, the arginine-glycine-aspartate (RGD) peptide can be cyclic (fKRGD).

Both the salt or acid form of a polymer described herein (e.g., the polymer obtained from polyglutamic acid and/or salt and an amino acid) can be used as starting material for forming the polymer conjugate with the targeting agent. In one embodiment, the targeting agent preferably in the presence of a coupling agent (e.g, DCC) and a catalyst (e.g, DMAP), can be reacted with the polymer obtained from polyglutamic acid and/or salt and an amino acid in a solvent (e.g, an aprotic solvent such as DMF). After formation of the polymer conjugate, any free amount of agent not covalently bonded to the polymer may also be measured. For example, thin layer chromatography (TLC) may be used to confirm the substantial absence of any free targeting agent. Suitable methods known to those skilled in the art can be used to isolate and/or purify the polymer conjugate (e.g., lypholization).

In an embodiment, a polymer comprising at least one recurring unit selected from formula (I), formula (II), and formula (III) (e.g., the polymer obtained from polyglutamic acid and/or salt and an amino acid) can be conjugated to the magnetic resonance imaging agent that includes a Gd(III) compound. One method for forming the magnetic resonance imaging agent is by reacting a paramagnetic metal with the polymer conjugate comprising the polydentate ligand. Paramagnetic metals include Gd(III), Indium-111, and Yttrium-88. For example, a polymer conjugate comprising DTPA can be treated with Gd(III) in a buffer solution for a period of several hours. Suitable methods known to those skilled in the art can be used to isolate and/or purify the polymer conjugate. For instance, the resulting reaction solution can be dialyzed in water using a cellulose membrane and the polymer can be lyophilized and isolated. The amount of paramagnetic metal may be quantified by inductively coupled plasma-optical emission spectroscopy (ICP-OES) measurement.

In one embodiment, a polymer comprising at least one recurring unit selected from formula (I), formula (II), and formula (III) (e.g., the polymer obtained from polyglutamic acid and/or salt and an amino acid) can be conjugated to polyethylene glycol. In one method, polyethylene glycol, preferably in the presence of a coupling agent (e.g, DCC) and a catalyst (e.g, DMAP), can be reacted with the polymer obtained from polyglutamic acid and/or salt and an amino acid in a solvent (e.g, an aprotic solvent such as DMF). Progress of the reaction can be measured by any suitable method such as TLC. The resulting polymer conjugate can be purified using methods known to those skilled in the art such as dialysis.

The polymer conjugates may be used to deliver the platinum compound, imaging agent, targeting agent, magnetic resonance imaging agent and/or the anti-cancer drug to a selected tissue. For example, polymer conjugates comprising the Texas Red dye may be used to deliver an imaging agent to a selected tissue. In one embodiment, the polymer conjugates comprising at least one recurring unit of the formulae (I), (II), and (III) can be used to treat or ameliorate a disease or condition such as cancer. In an embodiment, the polymer conjugates described herein can be used to diagnose a disease or condition (e.g., cancer). In yet one more embodiment, the polymer conjugates described herein can be used to image a portion of tissue. In some embodiments, the disease or condition can be a cancer such as lung cancer, breast cancer, colon cancer, ovarian cancer, prostate cancer, and melanoma. In an embodiment, the disease or condition can be a tumor selected from lung tumor, breast tumor, colon tumor, ovarian tumor, prostate tumor, and melanoma tumor. In some embodiments, the tissue being imaged can be tissue from lung tumor, breast tumor, colon tumor, ovarian tumor, prostate tumor, and/or melanoma tumor.

The polymers described above may be formed into nanoparticles in aqueous solution. Polymer conjugates (e.g., comprising a polymer and a drug and, optionally, other agent(s) as described herein) may be formed into nanoparticles in a similar manner. Such nanoparticles may be used to preferentially deliver a drug to a selected tissue.

An embodiment provides a composition that can include the polymer conjugate described herein and at least one selected from the group consisting of a pharmaceutically acceptable excipient, a carrier, and a diluent. In some embodiments, prodrugs, metabolites, stereoisomers, hydrates, solvates, polymorphs, and pharmaceutically acceptable salts of the compounds disclosed herein (e.g., the platinum-containing polymer conjugate and/or the agent that it comprises) are provided.

A "prodrug" refers to an agent that is converted into the parent drug *in vivo*. Prodrugs are often useful because, in some situations, they may be easier to administer than the parent drug. They may, for instance, be bioavailable by oral administration whereas the parent is not. The prodrug may also have improved solubility in pharmaceutical compositions over the parent drug. An example of a prodrug would be a compound which is administered as an ester (the "prodrug") to facilitate transmittal across a cell membrane where water solubility is detrimental to mobility but which then is metabolically hydrolyzed to the carboxylic acid, the active entity, once inside the cell where water-solubility is beneficial. A further example of a prodrug might be a short peptide (polyaminoacid) bonded to an acid group where the peptide is metabolized to reveal the active moiety. Conventional procedures for the selection and preparation of suitable prodrug derivatives are described, for example, in Design of Prodrugs, (ed. H. Bundgaard, Elsevier, 1985).

The term "pro-drug ester" refers to derivatives of the compounds disclosed herein formed by the addition of any of several ester-forming groups that are hydrolyzed under physiological conditions. Examples of pro-drug ester groups include pivaloyloxymethyl, acetoxymethyl, phthalidyl, indanyl and methoxymethyl, as well as other such groups known in the art, including a (5-R-2-oxo-1,3-dioxolen-4-yl)methyl group. Other examples of pro-drug ester groups can be found in, for example, T. Higuchi and V. Stella, in "Pro-drugs as Novel Delivery Systems", Vol. 14, A.C.S. Symposium Series, American Chemical Society (1975); and "Bioreversible Carriers in Drug Design: Theory and Application", edited by E. B. Roche, Pergamon Press: New York, 14-21 (1987) (providing examples of esters useful as prodrugs for compounds containing carboxyl groups).

The term "pharmaceutically acceptable salt" refers to a salt of a compound that does not cause significant irritation to an organism to which it is administered and does not abrogate the biological activity and properties of the compound. In some embodiments, the salt is an acid addition salt of the compound. Pharmaceutical salts can be obtained by reacting a compound with inorganic acids such as hydrohalic acid (e.g., hydrochloric acid or hydrobromic acid), sulfuric acid, nitric acid, phosphoric acid. Pharmaceutical salts can also be obtained by reacting a compound with an organic acid such as aliphatic or aromatic carboxylic or sulfonic acids, for example acetic, succinic, lactic, malic, tartaric, citric, ascorbic, nicotinic, methanesulfonic, ethanesulfonic, p-toluensulfonic, salicylic or naphthalenesulfonic acid. Pharmaceutical salts can also be obtained by reacting a compound with a base to form a salt such as an ammonium salt, an alkali metal salt, such as a sodium or a potassium salt, an alkaline earth metal salt, such as a calcium or a magnesium salt, a salt of organic bases such as dicyclohexylamine, N-methyl-D-glucamine, tris(hydroxymethyl)methylamine, C₁-C₇ alkylamine, cyclohexylamine, triethanolamine, ethylenediamine, and salts with amino acids such as arginine, lysine.

If the manufacture of pharmaceutical formulations involves intimate mixing of the pharmaceutical excipients and the active ingredient in its salt form, then it may be desirable to use pharmaceutical excipients which are non-basic, that is, either acidic or neutral excipients.

In various embodiments, the compounds disclosed herein (e.g., the platinum-containing polymer conjugate and/or the agent that it comprises) can be used alone, in combination with other compounds disclosed herein, or in combination with one or more other agents active in the therapeutic areas described herein.

In another aspect, the present disclosure relates to a pharmaceutical composition comprising one or more physiologically acceptable surface active agents, carriers, diluents, excipients, smoothing agents, suspension agents, film forming substances, and coating assistants, or a combination thereof; and a compound (e.g., the platinum-containing polymer conjugate and/or the agent that it comprises) disclosed herein. Acceptable carriers or diluents for therapeutic use are well known in the pharmaceutical art, and are described, for example, in Remington's Pharmaceutical Sciences, 18th Ed., Mack Publishing Co., Easton, PA (1990). Preservatives, stabilizers, dyes, sweeteners, fragrances, flavoring agents may be provided in the pharmaceutical composition. For example, sodium benzoate, ascorbic acid and esters of p-hydroxybenzoic acid may be added as preservatives. In addition, antioxidants and suspending agents may be used. In various embodiments, alcohols, esters, sulfated aliphatic alcohols may be used as surface active agents; sucrose, glucose, lactose, starch, crystallized cellulose, mannitol, light anhydrous silicate, magnesium aluminate, magnesium metasilicate aluminate, synthetic aluminum silicate, calcium carbonate, sodium acid carbonate, calcium hydrogen phosphate, calcium carboxymethyl cellulose may be used as excipients; magnesium stearate, talc, hardened oil may be used as smoothing agents; coconut oil, olive oil, sesame oil, peanut oil, soya may be used as suspension agents or lubricants; cellulose acetate phthalate as a derivative of a carbohydrate such as cellulose or sugar, or methylacetate-methacrylate copolymer as a derivative of polyvinyl may be used as suspension agents; and plasticizers such as ester phthalates may be used as suspension agents.

The term "pharmaceutical composition" refers to a mixture of a compound disclosed herein (e.g., the platinum-containing polymer conjugate and/or the agent that it comprises) with other chemical components, such as diluents or carriers. The pharmaceutical composition facilitates administration of the compound to an organism. Multiple techniques of administering a compound exist in the art including oral, injection, aerosol, parenteral, and topical administration. Pharmaceutical compositions can also be obtained by reacting compounds with inorganic or organic acids such as hydrochloric acid, hydrobromic acid, sulfuric acid, nitric acid, phosphoric acid, methanesulfonic acid, ethanesulfonic acid, p-toluenesulfonic acid, salicylic acid .

The term "carrier" refers to a chemical compound that facilitates the incorporation of a compound into cells or tissues. For example dimethyl sulfoxide (DMSO) is a commonly utilized carrier as it facilitates the uptake of many organic compounds into the cells or tissues of an organism.

The term "diluent" refers to chemical compounds diluted in water that will dissolve the compound of interest (e.g., the platinum-containing polymer conjugate and/or the agent that it comprises) as well as stabilize the biologically active form of the compound. Salts dissolved in buffered solutions are utilized as diluents in the art. One commonly used buffered solution is phosphate buffered saline because it mimics the salt conditions of human blood. Since buffer salts can control the pH of a solution at low concentrations, a buffered diluent rarely modifies the biological activity of a compound. The term "physiologically acceptable" refers to a carrier or diluent that does not abrogate the biological activity and properties of the compound.

The pharmaceutical compositions described herein can be administered to a human patient *per se*, or in pharmaceutical compositions where they are mixed with other active ingredients, as in combination therapy, or suitable carriers or excipient(s). Techniques for formulation and administration of the compounds of the instant application may be found in "Remington's Pharmaceutical Sciences," Mack Publishing Co., Easton, PA, 18th edition, 1990.

Suitable routes of administration may, for example, include oral, rectal, transmucosal, topical, or intestinal administration; parenteral delivery, including intramuscular, subcutaneous, intravenous, intramedullary injections, as well as intrathecal, direct intraventricular, intraperitoneal, intranasal, or intraocular injections. In some embodiments, the compounds (e.g., the platinum-containing polymer conjugate and/or the agent that it comprises) can also be administered in sustained or controlled release dosage forms, including depot injections, osmotic pumps, pills, transdermal (including electrotransport) patches for prolonged and/or timed, pulsed administration at a predetermined rate.

The pharmaceutical compositions may be manufactured in a manner that is itself known, *e.g*., by means of conventional mixing, dissolving, granulating, dragee-making, levigating, emulsifying, encapsulating, entrapping or tabletting processes.

Pharmaceutical compositions may be formulated in a conventional manner using one or more physiologically acceptable carriers comprising excipients and auxiliaries which facilitate processing of the active compounds into preparations which can be used pharmaceutically. Proper formulation is dependent upon the route of administration chosen. Any of the well-known techniques, carriers, and excipients may be used as suitable and as understood in the art; *e.g*., in Remington's Pharmaceutical Sciences, above.

Injectables can be prepared in conventional forms, either as liquid solutions or suspensions, solid forms suitable for solution or suspension in liquid prior to injection, or as emulsions. Suitable excipients are, for example, water, saline, dextrose, mannitol, lactose, lecithin, albumin, sodium glutamate, cysteine hydrochloride. In addition, if desired, the injectable pharmaceutical compositions may contain minor amounts of nontoxic auxiliary substances, such as wetting agents, pH buffering agents. Physiologically compatible buffers include Hanks's solution, Ringer's solution, or physiological saline buffer. If desired, absorption enhancing preparations (for example, liposomes), may be utilized.

For transmucosal administration, penetrants appropriate to the barrier to be permeated may be used in the formulation.

Pharmaceutical formulations for parenteral administration, *e.g*., by bolus injection or continuous infusion, include aqueous solutions of the active compounds in water-soluble form. Additionally, suspensions of the active compounds may be prepared as appropriate oily injection suspensions. Suitable lipophilic solvents or vehicles include fatty oils such as sesame oil, or other organic oils such as soybean, grapefruit or almond oils, or synthetic fatty acid esters, such as ethyl oleate or triglycerides, or liposomes. Aqueous injection suspensions may contain substances which increase the viscosity of the suspension, such as sodium carboxymethyl cellulose, sorbitol, or dextran. Optionally, the suspension may also contain suitable stabilizers or agents that increase the solubility of the compounds to allow for the preparation of highly concentrated solutions. Formulations for injection may be presented in unit dosage form, *e.g*., in ampoules or in multi-dose containers, with an added preservative. The compositions may take such forms as suspensions, solutions or emulsions in oily or aqueous vehicles, and may contain formulatory agents such as suspending, stabilizing and/or dispersing agents. Alternatively, the active ingredient may be in powder form for constitution with a suitable vehicle, *e.g*., sterile pyrogen-free water, before use.

For oral administration, the compounds (e.g., the platinum-containing polymer conjugate and/or the agent that it comprises) can be formulated readily by combining the active compounds with pharmaceutically acceptable carriers well known in the art. Such carriers enable the compounds of the invention to be formulated as tablets, pills, dragees, capsules, liquids, gels, syrups, slurries, suspensions for oral ingestion by a patient to be treated. Pharmaceutical preparations for oral use can be obtained by combining the active compounds with solid excipient, optionally grinding a resulting mixture, and processing the mixture of granules, after adding suitable auxiliaries, if desired, to obtain tablets or dragee cores. Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethyl-cellulose, sodium carboxymethylcellulose, and/or polyvinylpyrrolidone (PVP). If desired, disintegrating agents may be added, such as the cross-linked polyvinyl pyrrolidone, agar, or alginic acid or a salt thereof such as sodium alginate. Dragee cores are provided with suitable coatings. For this purpose, concentrated sugar solutions may be used, which may optionally contain gum arabic, talc, polyvinyl pyrrolidone, carbopol gel, polyethylene glycol, and/or titanium dioxide, lacquer solutions, and suitable organic solvents or solvent mixtures. Dyestuffs or pigments may be added to the tablets or dragee coatings for identification or to characterize different combinations of active compound doses. For this purpose, concentrated sugar solutions may be used, which may optionally contain gum arabic, talc, polyvinyl pyrrolidone, carbopol gel, polyethylene glycol, and/or titanium dioxide, lacquer solutions, and suitable organic solvents or solvent mixtures. Dyestuffs or pigments may be added to the tablets or dragee coatings for identification or to characterize different combinations of active compound doses.

Pharmaceutical preparations which can be used orally include push-fit capsules made of gelatin, as well as soft, sealed capsules made of gelatin and a plasticizer, such as glycerol or sorbitol. The push-fit capsules can contain the active ingredients in admixture with filler such as lactose, binders such as starches, and/or lubricants such as talc or magnesium stearate and, optionally, stabilizers. In soft capsules, the active compounds may be dissolved or suspended in suitable liquids, such as fatty oils, liquid paraffin, or liquid polyethylene glycols. In addition, stabilizers may be added. All formulations for oral administration should be in dosages suitable for such administration.

For buccal administration, the compositions may take the form of tablets or lozenges formulated in conventional manner.

For administration by inhalation, the compounds (e.g., the platinum-containing polymer conjugate and/or the agent that it comprises) are conveniently delivered in the form of an aerosol spray presentation from pressurized packs or a nebulizer, with the use of a suitable propellant, *e.g*., dichlorodifluoromethane, trichlorofluoromethane, dichlorotetrafluoroethane, carbon dioxide or other suitable gas. In the case of a pressurized aerosol the dosage unit may be determined by providing a valve to deliver a metered amount. Capsules and cartridges of, *e.g*., gelatin for use in an inhaler or insufflator may be formulated containing a powder mix of the compound and a suitable powder base such as lactose or starch.

Further disclosed herein are various pharmaceutical compositions well known in the pharmaceutical art for uses that include intraocular, intranasal, and intraauricular delivery. Suitable penetrants for these uses are generally known in the art. Pharmaceutical compositions for intraocular delivery include aqueous ophthalmic solutions of the active compounds in water-soluble form, such as eyedrops, or in gellan gum (Shedden et al., Clin. Ther., 23(3):440-50 (2001)) or hydrogels (Mayer et al., Ophthalmologica, 210(2):101-3 (1996)); ophthalmic ointments; ophthalmic suspensions, such as microparticulates, drug-containing small polymeric particles that are suspended in a liquid carrier medium (Joshi, A., J. Ocul. Pharmacol., 10(1):29-45 (1994)), lipid-soluble formulations (Alm et al., Prog. Clin. Biol. Res., 312:447-58 (1989)), and microspheres (Mordenti, Toxicol. Sci., 52(1):101-6 (1999)); and ocular inserts. Such suitable pharmaceutical formulations are most often and preferably formulated to be sterile, isotonic and buffered for stability and comfort. Pharmaceutical compositions for intranasal delivery may also include drops and sprays often prepared to simulate in many respects nasal secretions to ensure maintenance of normal ciliary action. As disclosed in Remington's Pharmaceutical Sciences, 18th Ed., Mack Publishing Co., Easton, PA (1990), and well-known to those skilled in the art, suitable formulations are most often and preferably isotonic, slightly buffered to maintain a pH of 5.5 to 6.5, and most often and preferably include antimicrobial preservatives and appropriate drug stabilizers. Pharmaceutical formulations for intraauricular delivery include suspensions and ointments for topical application in the ear. Common solvents for such aural formulations include glycerin and water.

The compounds (e.g., the platinum-containing polymer conjugate and/or the agent that it comprises) may also be formulated in rectal compositions such as suppositories or retention enemas, *e.g*., containing conventional suppository bases such as cocoa butter or other glycerides.

In addition to the formulations described previously, the compounds (e.g., the platinum-containing polymer conjugate and/or the agent that it comprises) may also be formulated as a depot preparation. Such long acting formulations may be administered by implantation (for example subcutaneously or intramuscularly) or by intramuscular injection. Thus, for example, the compounds may be formulated with suitable polymeric or hydrophobic materials (for example as an emulsion in an acceptable oil) or ion exchange resins, or as sparingly soluble derivatives, for example, as a sparingly soluble salt.

For hydrophobic compounds, a suitable pharmaceutical carrier may be a cosolvent system comprising benzyl alcohol, a nonpolar surfactant, a water-miscible organic polymer, and an aqueous phase. A common cosolvent system used is the VPD co-solvent system, which is a solution of 3% w/v benzyl alcohol, 8% w/v of the nonpolar surfactant Polysorbate 80™, and 65% w/v polyethylene glycol 300, made up to volume in absolute ethanol. Naturally, the proportions of a co-solvent system may be varied considerably without destroying its solubility and toxicity characteristics. Furthermore, the identity of the co-solvent components may be varied: for example, other low-toxicity nonpolar surfactants may be used instead of POLYSORBATE 80™; the fraction size of polyethylene glycol may be varied; other biocompatible polymers may replace polyethylene glycol, *e.g*., polyvinyl pyrrolidone; and other sugars or polysaccharides may substitute for dextrose.

Alternatively, other delivery systems for hydrophobic pharmaceutical compounds may be employed. Liposomes and emulsions are well known examples of delivery vehicles or carriers for hydrophobic drugs. Certain organic solvents such as dimethylsulfoxide also may be employed, although usually at the cost of greater toxicity. Additionally, the compounds may be delivered using a sustained-release system, such as semipermeable matrices of solid hydrophobic polymers containing the therapeutic agent. Various sustained-release materials have been established and are well known by those skilled in the art. Sustained-release capsules may, depending on their chemical nature, release the compounds for a few hours or weeks up to over 100 days. Depending on the chemical nature and the biological stability of the therapeutic reagent, additional strategies for protein stabilization may be employed.

Agents intended to be administered intracellularly may be administered using techniques well known to those of ordinary skill in the art. For example, such agents may be encapsulated into liposomes. All molecules present in an aqueous solution at the time of liposome formation are incorporated into the aqueous interior. The liposomal contents are both protected from the external micro-environment and, because liposomes fuse with cell membranes, are efficiently delivered into the cell cytoplasm. The liposome may be coated with a tissue-specific antibody. The liposomes will be targeted to and taken up selectively by the desired organ. Alternatively, small hydrophobic organic molecules may be directly administered intracellularly.

Additional therapeutic or diagnostic agents may be incorporated into the pharmaceutical compositions. Alternatively or additionally, pharmaceutical compositions may be combined with other compositions that contain other therapeutic or diagnostic agents.

The compounds (e.g., the platinum-containing polymer conjugate and/or the agent that it comprises) or pharmaceutical compositions thereof may be administered to the patient by any suitable means. Examples of methods of administration include, among others, (a) administration though oral pathways, which administration includes administration in capsule, tablet, granule, spray, syrup, or other such forms; (b) administration through non-oral pathways such as rectal, vaginal, intraurethral, intraocular, intranasal, or intraauricular, which administration includes administration as an aqueous suspension, an oily preparation or as a drip, spray, suppository, salve, ointment; (c) administration via injection, subcutaneously, intraperitoneally, intravenously, intramuscularly, intradermally, intraorbitally, intracapsularly, intraspinally, intrasternally, including infusion pump delivery; (d) administration locally such as by injection directly in the renal or cardiac area, e.g., by depot implantation; as well as (e) administration topically; as deemed appropriate by those of skill in the art for bringing the active compound into contact with living tissue.

Pharmaceutical compositions suitable for administration include compositions where the active ingredients are contained in an amount effective to achieve its intended purpose. The effective amount of the compounds disclosed herein required as a dose will depend on the route of administration, the type of animal, including human, being treated, and the physical characteristics of the specific animal under consideration. The dose can be tailored to achieve a desired effect, but will depend on such factors as weight, diet, concurrent medication and other factors which those skilled in the medical arts will recognize. More specifically, an effective amount means an amount of compound effective to prevent, alleviate or ameliorate symptoms of disease or prolong the survival of the subject being treated. Determination of an effective amount is well within the capability of those skilled in the art, especially in light of the detailed disclosure provided herein.

As will be readily apparent to one skilled in the art, the useful in vivo dosage to be administered and the particular mode of administration will vary depending upon the age, weight and mammalian species treated, the particular compounds employed, and the specific use for which these compounds are employed. The determination of effective dosage levels, that is the dosage levels necessary to achieve the desired result, can be accomplished by one skilled in the art using routine pharmacological methods. Typically, human clinical applications of products are commenced at lower dosage levels, with dosage level being increased until the desired effect is achieved. Alternatively, acceptable *in vitro* studies can be used to establish useful doses and routes of administration of the compositions identified by the present methods using established pharmacological methods.

In non-human animal studies, applications of potential products are commenced at higher dosage levels, with dosage being decreased until the desired effect is no longer achieved or adverse side effects disappear. The dosage may range broadly, depending upon the desired effects and the therapeutic indication. Typically, dosages may be between about 10 microgram/kg and 100 mg/kg body weight, preferably between about 100 microgram/kg and 10 mg/kg body weight. Alternatively dosages may be based and calculated upon the surface area of the patient, as understood by those of skill in the art.

The exact formulation, route of administration and dosage for the pharmaceutical compositions described herein can be chosen by the individual physician in view of the patient's condition. (See *e.g*., Fingl et al. 1975, in "The Pharmacological Basis of Therapeutics", with particular reference to Ch. 1, p. 1). Typically, the dose range of the composition administered to the patient can be from about 0.5 to 1000 mg/kg of the patient's body weight. The dosage may be a single one or a series of two or more given in the course of one or more days, as is needed by the patient. In instances where human dosages for compounds have been established for at least some condition, the present invention will use those same dosages, or dosages that are between about 0.1% and 500%, more preferably between about 25% and 250% of the established human dosage. Where no human dosage is established, as will be the case for newly-discovered pharmaceutical compositions, a suitable human dosage can be inferred from ED₅₀ or ID₅₀ values, or other appropriate values derived from *in vitro* or *in vivo* studies, as qualified by toxicity studies and efficacy studies in animals.

It should be noted that the attending physician would know how to and when to terminate, interrupt, or adjust administration due to toxicity or organ dysfunctions. Conversely, the attending physician would also know to adjust treatment to higher levels if the clinical response were not adequate (precluding toxicity). The magnitude of an administrated dose in the management of the disorder of interest will vary with the severity of the condition to be treated and to the route of administration. The severity of the condition may, for example, be evaluated, in part, by standard prognostic evaluation methods. Further, the dose and perhaps dose frequency, will also vary according to the age, body weight, and response of the individual patient. A program comparable to that discussed above may be used in veterinary medicine.

Although the exact dosage will be determined on a drug-by-drug basis, in most cases, some generalizations regarding the dosage can be made. The daily dosage regimen for an adult human patient may be, for example, an oral dose of between 0.1 mg and 2000 mg of each active ingredient, preferably between 1 mg and 500 mg, e.g. 5 to 200 mg. In other embodiments, an intravenous, subcutaneous, or intramuscular dose of each active ingredient of between 0.01 mg and 100 mg, preferably between 0.1 mg and 60 mg, e.g. 1 to 40 mg is used. In cases of administration of a pharmaceutically acceptable salt, dosages may be calculated as the free base. In some embodiments, the composition is administered 1 to 4 times per day. Alternatively the compositions of the invention may be administered by continuous intravenous infusion, preferably at a dose of each active ingredient up to 1000 mg per day. As will be understood by those of skill in the art, in certain situations it may be necessary to administer the compounds disclosed herein in amounts that exceed, or even far exceed, the above-stated, preferred dosage range in order to effectively and aggressively treat particularly aggressive diseases or infections. In some embodiments, the compounds will be administered for a period of continuous therapy, for example for a week or more, or for months or years.

Dosage amount and interval may be adjusted individually to provide plasma levels of the active moiety which are sufficient to maintain the modulating effects, or minimal effective concentration (MEC). The MEC will vary for each compound but can be estimated from in vitro data. Dosages necessary to achieve the MEC will depend on individual characteristics and route of administration. However, HPLC assays or bioassays can be used to determine plasma concentrations.

Dosage intervals can also be determined using MEC value. Compositions should be administered using a regimen which maintains plasma levels above the MEC for 10-90% of the time, preferably between 30-90% and most preferably between 50-90%.

In cases of local administration or selective uptake, the effective local concentration of the drug may not be related to plasma concentration.

The amount of composition administered may be dependent on the subject being treated, on the subject's weight, the severity of the affliction, the manner of administration and the judgment of the prescribing physician.

Compounds disclosed herein (e.g., the platinum-containing polymer conjugate and/or the agent that it comprises) can be evaluated for efficacy and toxicity using known methods. For example, the toxicology of a particular compound, or of a subset of the compounds, sharing certain chemical moieties, may be established by determining *in vitro* toxicity towards a cell line, such as a mammalian, and preferably human, cell line. The results of such studies are often predictive of toxicity in animals, such as mammals, or more specifically, humans. Alternatively, the toxicity of particular compounds in an animal model, such as mice, rats, rabbits, or monkeys, may be determined using known methods. The efficacy of a particular compound may be established using several recognized methods, such as *in vitro* methods, animal models, or human clinical trials. Recognized *in vitro* models exist for nearly every class of condition, including cancer, cardiovascular disease, and various immune dysfunction. Similarly, acceptable animal models may be used to establish efficacy of chemicals to treat such conditions. When selecting a model to determine efficacy, the skilled artisan can be guided by the state of the art to choose an appropriate model, dose, and route of administration, and regime. Of course, human clinical trials can also be used to determine the efficacy of a compound in humans.

The compositions may, if desired, be presented in a pack or dispenser device which may contain one or more unit dosage forms containing the active ingredient. The pack may for example comprise metal or plastic foil, such as a blister pack. The pack or dispenser device may be accompanied by instructions for administration. The pack or dispenser may also be accompanied with a notice associated with the container in form prescribed by a governmental agency regulating the manufacture, use, or sale of pharmaceuticals, which notice is reflective of approval by the agency of the form of the drug for human or veterinary administration. Such notice, for example, may be the labeling approved by the U.S. Food and Drug Administration for prescription drugs, or the approved product insert. Compositions comprising a compound of the invention formulated in a compatible pharmaceutical carrier may also be prepared, placed in an appropriate container, and labeled for treatment of an indicated condition.

Polymers and copolymers comprising a recurring unit of the formula (I) may have many different uses. The polymer conjugates of the invention may be used in a method of treating or ameliorating a disease or condition comprising administering an effective amount of one or more polymer conjugates described herein or the pharmaceutical composition described herein to a mammal in need thereof. Another embodiment provides an effective amount of one or more polymer conjugates described herein or the pharmaceutical composition described herein for treating or ameliorating a disease or condition. In an embodiment, the disease or condition is selected from lung tumor, breast tumor, colon tumor, ovarian tumor, prostate tumor, and melanoma tumor. In an embodiment, the disease or condition is selected from lung cancer, breast cancer, colon cancer, ovarian cancer, prostate cancer, and melanoma. The polymer conjugates of the invention may be used in a method of treating or ameliorating a disease or condition comprising administering an effective amount of a the polymer conjugates described herein (e.g., a polymer conjugate comprising units of formula (I), formula (II), and/or formula (III)) and further comprising administrating an effective amount of an agent that can include rosizitaglone. The polymer conjugates of the invention may be used in a method of treating or ameliorating a disease or condition comprising administering an effective amount of a the polymer conjugates described herein (e.g., a polymer conjugate comprising units of formula (I), formula (II), and/or formula (III) and further comprising administering an effective amount of an agent that can activate PPARγ, for example, rosizitaglone and pioglitazone.

The administration of the polymer conjugate and the agent that can activate PPARγ may be carried out in various ways. For example, in some embodiments, the polymer conjugate can be administering before the agent that can activate PPARγ (e.g., rosizitaglone or pioglitazone). In other embodiments, the polymer conjugate can be administering before the agent that can activate PPARγ. In still other embodiments, the polymer conjugate can be administering at about the same time as the agent that can activate PPARγ. In an embodiment, the polymer conjugate and the agent that can activate PPARγ, for example rosizitaglone and pioglitazone, can be administered in a single dosage form. In another embodiment, the polymer conjugate and the agent that can activate PPARγ can be administered in a separate dosage forms. Furthermore, the polymer conjugate such as those described herein and the agent that can activate PPARγ can be administered by the same method, for example, both orally. Also, the polymer conjugate such as those described herein and the agent that can activate PPARγ can be administered by different methods such as one can be administered orally and the other can be administered intravenously.

The polymer conjugates of the invention may be used in a method of diagnosing a disease or condition comprising administering an effective amount of one or more polymer conjugates described herein or the pharmaceutical composition described herein to a mammal in need thereof. Another embodiment provides an effective amount of one or more polymer conjugates described herein or the pharmaceutical composition described herein for diagnosing a disease or condition. In an embodiment, the disease or condition is selected from lung tumor, breast tumor, colon tumor, ovarian tumor, prostate tumor, and melanoma tumor. In an embodiment, the disease or condition is selected from lung cancer, breast cancer, colon cancer, ovarian cancer, prostate cancer, and melanoma.

The polymer conjugates of the invention may be used in a method of imaging a portion of tissue comprising contacting a portion of tissue with an effective amount of one or more polymer conjugates described herein or the pharmaceutical composition described herein. Another embodiment provides an effective amount of one or more polymer conjugates described herein or the pharmaceutical composition described herein for imaging a portion of tissue. In some embodiments, the tissue being imaged can be tissue from lung tumor, breast tumor, colon tumor, ovarian tumor, prostate tumor, and/or melanoma tumor.

### EXAMPLES

The following examples are provided for the purposes of further describing the embodiments described herein.

### Materials

Poly-L-glutamate sodium salts with different molecular weights (average molecular weights of 41,400 (PGA(97k)), 17,600 (PGA(44k)), 16,000 (PGA(32k)), and 10,900 (PGA(21k)) daltons based on multi-angle light scattering (MALS)); N-(3-dimethylaminopropyl)-N'-ethylcarbodiimide hydrochloride (EDC); hydroxybenzotriazole (HOBt); pyridine; 4-dimethylaminopyridine (DMAP); N,N'-dimethylformamide (DMF); gadolinium-acetate; chloroform; camptothecin, and sodium bicarbonate were purchased from Sigma-Aldrich Chemical company. Poly-L-glutamate was converted into poly-L-glutamic acid using 2 N hydrochloric acid solution. Trifluoroacetic acid (TFA) was purchased from Bioscience. L-glutamic acid di-t-butyl ester hydrochloride (H-Glu(OtBu)-OtBu•HCl), N-α-CBZ-L-glutamic acid α-benzyl ester (Z-Glu-OBzl) were purchased from Novabiochem (La Jolla, CA). Paclitaxel and doxorubicin was purchased from PolyMed (Houston, TexasThe chemical p-NH₂-Bn-DPTA-penta-(*t*-Bu ester) was purchased from Macrocyclics (Dallas, Texas). ¹H NMR was obtained from Joel (400 MHz), and particle sizes were measured by ZetalPals (Brookhaven Instruments Corporation). Microwave chemistry was carried out in Biotage. Molecular weights of polymers were determined by size exclusion chromatography (SEC) combined with a multi-angle light scattering (MALS) (Wyatt Corporation) detector.

A poly-(γ-L-glutamyl-glutamine) were prepared from a polyglutamate sodium salt, according to the procedures described in U.S. Patent Publication No. 2007-0128118, filed December 1, 2006, which describes the synthesis of the polymers described therein (e.g, poly-(γ-L-glutamyl-glutamine), poly-(γ-L-aspartyl-glutamine), poly-(γ-L-glutamyl-glutamine)-poly-L-glutamic acid, and poly-(y-L- aspartyl-glutamine)-poly-L-glutamic acid. Average molecular weights of the polymers were determined using the system and conditions described below (hereinafter, referred to as the Heleos system with MALS detector).

### SEC-MALS Analysis Conditions:

| | |
|---|---|
| ■ HPLC system: | Agilent 1200 |
| ■ Column: | Shodex SB 806M HQ (exclusion limit for Pullulan is 20,000,000, particle size: 13 micron, size (mm) IDxLength; 8.0 x300) |
| ■ Mobile Phase: | 1xDPBS or 1% LiBr in DPBS (pH7.0) |
| ■ Flow Rate: | 1 ml/min |
| ■ MALS detector: | DAWN HELEOS from Wyatt |
| ■ DRI detector: | Optilab rEX from Wyatt |
| ■ On-line Viscometer: | ViscoStar from Wyatt |
| ■ Software: | ASTRA 5.1.9 from Wyatt |
| ■ Sample Concentration: | 1-2 mg/ml |
| ■ Injection volume: | 100 µl |

| | |
|---|---|
| dn/dc value of polymer: 0.185 was used in the measurement. BSA was used as a control before actual samples are run. | |

Sulforhodamine B dye for cytotoxic MTT test (cell viability) was purchased from Molecular Imaging Products Company (Michigan). Poly-(β-aspartyl-glutamine)-paclitaxel conjugates (PGA-21-G-paclitaxel-20 and PGA-32-G-paclitaxel-20) and poly-(γ-L-glutamyl-glutamine) were synthesized according to the procedures described in U.S. Patent Publication No. 2007-0128118. The content of paclitaxel in polymer-paclitaxel conjugates was estimated by UV/Vis spectrometry (Lambda Bio 40, PerkinElmer) based on a standard curve generated with known concentrations of paclitaxel in methanol (λ = 228 nm). Synthesis of poly-L-glutamate-paclitaxel conjugates (PGA-PTX) was carried out as reported in previous literature. See Li et al., "Complete regression of well-established tumors using a novel water-soluble poly(L-glutamic acid)-paclitaxel conjugate," Cancer Research 1998, 58, 2404-2409 .

### EXAMPLE 1

### Formulation of cisplatin using PGA-21-G-paclitaxel-20

PGA-21-G-paclitaxel-20 (92 mg) was dissolved in distilled water (3 mL). The mixture was then sonicated for 5 minutes. A solution of cisplatin (8 mg) in ethanol (0.4 mL) was added into the sample using a pipette. This mixture solution was sonicated for 5 minutes. The resulting mixture was freeze-dried and store at -20°C.

### EXAMPLE 2

### Formulation of cisplatin using PGA-G

Poly-(γ-L-glutamyl-glutamine) (100 mg) was dissolved in distilled water (3 mL). The mixture was then sonicated for 5 minutes. A solution of cisplatin (11 mg) in ethanol (0.4 mL) was added into the sample using a pipette. This mixture solution was sonicated for 5 minutes. The resulting mixture was freeze-dried and store at -20°C.

## Claims

1. A polymer conjugate comprising at least one recurring unit selected from formula (I), formula (II), and formula (III): 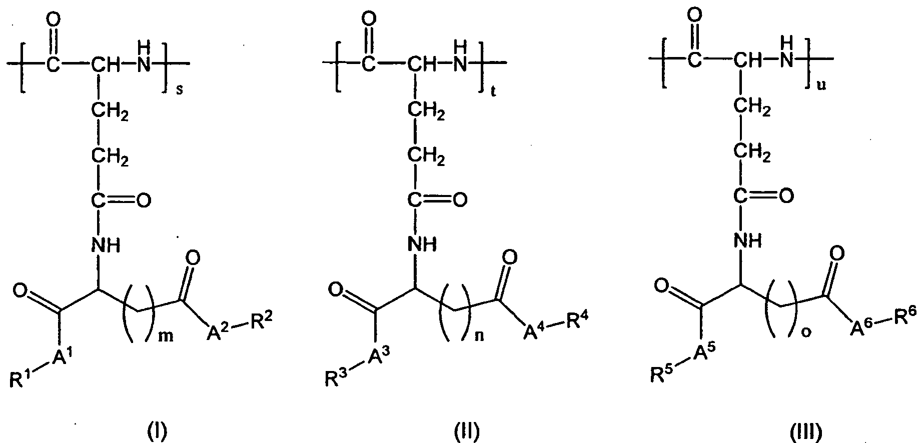 wherein:
A¹, A², A³, A⁴, A⁵ and A⁶ are each independently oxygen or NR⁷, wherein R⁷ is hydrogen or a C₁₋₄ alkyl;
R¹, R², R³, R⁴, R⁵ and R⁶ are each independently selected from the group consisting of a hydrogen, a C₁₋₁₀ alkyl group, a C₆₋₂₀ aryl group, an ammonium group, an alkali metal, a polydentate ligand, a polydentate ligand precursor with protected oxygen atoms, a group that comprises platinum, a group that comprises a drug, a group that comprises a targeting agent, a group that comprises an optical imaging agent, a group that comprises a magnetic resonance imaging agent, and a group that comprises a stabilizing agent;
wherein the polydentate ligand comprises: 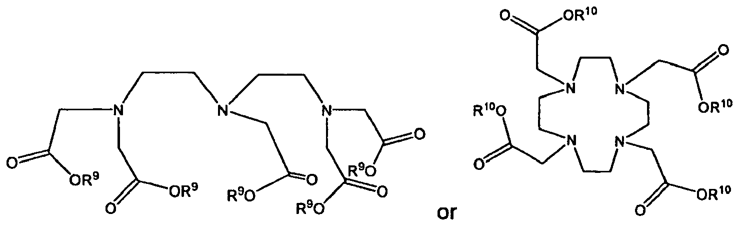 wherein each R⁹ is independently hydrogen, ammonium, or an alkali metal;
and
wherein each R¹⁰ is independently hydrogen, ammonium, or an alkali metal;
wherein the polydentate ligand precursor with protected oxygen atoms comprises: 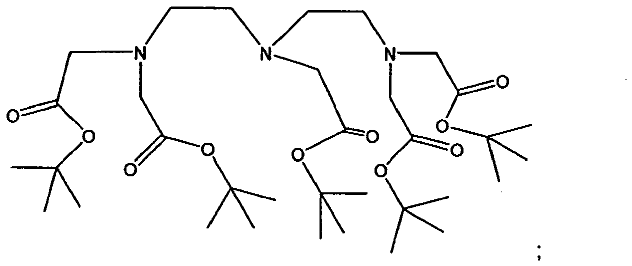 wherein the group that comprises platinum comprises cisplatin, carboplatin, or oxaliplatin, or wherein the recurring unit of formula (I) has the structure: 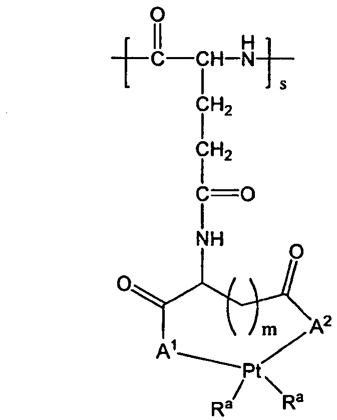 wherein each R^{a} is independently selected from the group consisting of monoalkyl amine, dialkylamine, monoaryl amine, and diaryl amine;
wherein the group that comprises a drug comprises an anticancer drug;
wherein the group that comprises the targeting agent is selected from the group consisting of an arginine-glycine-aspartate (RGD) peptide, fibronectin, folate, galactose, an apolipoprotein, insulin, transferrin, a fibroblast growth factor (FGF), an epidermal growth factor (EGF), and an antibody;
wherein the group that comprises an optical imaging agent is selected from the group consisting of an acridine dye, a coumarine dye, a rhodamine dye, a xanthene dye, a cyanine dye, and a pyrene dye;
wherein the group that comprises a magnetic resonance imaging agent
comprises: 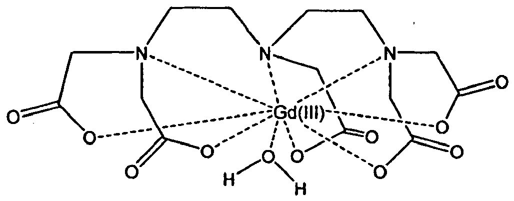 or 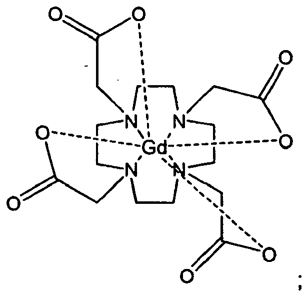 wherein the group that comprises a stabilizing agent comprises polyethylene glycol;
at least one of R¹, R², R³, R⁴, R⁵ and R⁶ is a group that comprises platinum; m, n, and o are each independently 1 or 2; and
s, t, and u are each independently 0 or ≥ 1, wherein s + t + u is ≥ 1.

2. The polymer conjugate of Claim 1, wherein s is 1 or greater; and t and u are 0.

3. The polymer conjugate of any one of Claims 1 to 2, wherein the group that comprises platinum is attached to both A¹ and A².

4. The polymer conjugate of Claim 3, wherein the recurring unit of formula (I) has the structure: 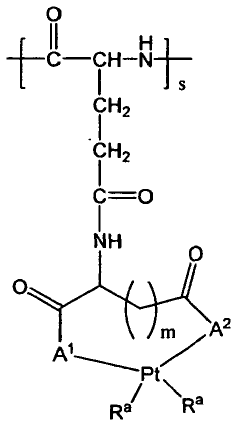 wherein each R^{a} is independently selected from the group consisting of monoalkyl amine, dialkylamine, monoaryl amine, and diaryl amine.

5. The polymer conjugate of Claim 3, wherein the recurring unit of formula (I) has the structure:

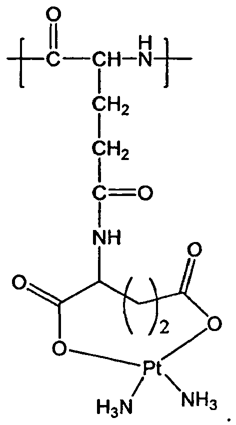

6. The polymer conjugate of Claim 1, wherein s + t is 2 or greater; and the group that comprises platinum is attached to A² and A³, or wherein s + t is 2 or greater; and the group that comprises platinum is attached to A² and A⁴, or wherein s + t is 2 or greater; and the group that comprises platinum is attached to A¹ and A³.

7. The polymer conjugate of Claim 1 or Claim 6, wherein
s + t is 2 or greater; u is 0; and at least one of R¹, R², R³ and R⁴ is the group that comprises platinum, and at least one of R¹, R², R³ and R⁴ is a group that comprises the drug, wherein the group that comprises platinum and the group that comprises the drug are not the same; or
s + t is 2 or greater; u is 0; and at least one of R¹, R², R³ and R⁴ is a group that comprises platinum, and at least one of R¹, R², R³ and R⁴ is selected from the group consisting of the polydentate ligand, the polydentate ligand precursor with protected oxygen atoms, the group that comprises a targeting agent, the group that comprises an optical imaging agent, the group that comprises a magnetic resonance imaging agent, and the group that comprises a stabilizing agent.

8. The polymer conjugate of Claim 1, wherein s + t + u is 3 or greater.

9. The polymer conjugate of Claim 8, wherein R⁵ and R⁶ are each independently selected from the group consisting of a hydrogen, a C₁₋₁₀ alkyl group, a C₆₋₂₀ aryl group, an ammonium group and an alkali metal.

10. The polymer conjugate of any one of Claims 1 to 9, further comprising a recurring unit of formula (IV): 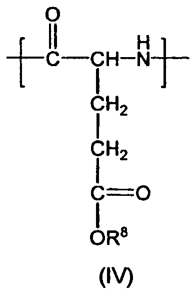 wherein R⁸ is hydrogen, ammonium, or an alkali metal.

11. The polymer conjugate of any one of Claims 1 to 10, wherein the polymer conjugate comprises a total amount of the platinum in the range of about 0.5% to about 50% (weight/weight) based on the mass ratio of the platinum to the polymer conjugate.

12. The polymer conjugate of any one of Claims 1 to 11, wherein the anticancer drug is selected from the group consisting of a taxane, camptotheca, and anthracycline.

13. The polymer conjugate of any one of Claims 1 to 12, wherein the anticancer drug is selected from the group consisting of paclitaxel, docetaxel, camptothecin and doxorubicin.

14. The polymer conjugate of any one of Claims 1 to 13, further comprising an agent that activates PPARγ.

15. A pharmaceutical composition comprising the polymer conjugate of any one of Claims 1 to 14 and at least one selected from a pharmaceutically acceptable excipient, a carrier, and a diluent.

16. A method of making the polymer conjugate of Claim 1 comprising
dissolving or partially dissolving a polymeric reactant comprising a recurring unit of formula (V) in a solvent to form a dissolved or partially dissolved polymeric reactant; 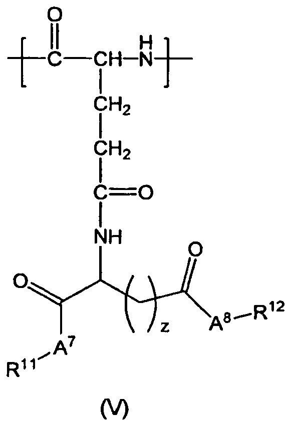 wherein:
z is independently 1 or 2;
A⁷ and A⁸ are oxygen; and
R¹¹ and R¹² are each independently selected from the group consisting of hydrogen, ammonium, and an alkali metal; and
reacting the dissolved or partially dissolved polymeric reactant with a second reactant, wherein the second reactant comprises the group comprising platinum.

17. Use of the polymer conjugate of any one of Claims 1 to 14 or the pharmaceutical composition of Claim 15 in the preparation of a medicament for treating or ameliorating a disease or condition, or in the preparation of a medicament for diagnosing a disease or condition.

18. A polymer conjugate of any one of Claims 1-14 or a pharmaceutical composition of Claim 15 for use in treating or ameliorating a disease or condition, or for use in diagnosing a disease or condition.

19. The use of Claim 17 in the preparation of a medicament for treating or ameliorating a disease or condition, or the polymer conjugate or pharmaceutical composition of Claim 18 for use in treating or ameliorating a disease or condition, further comprising administering one or more compounds selected from a group that comprises rosizitaglone and a group that comprises pioglitazone.

20. The use of Claim 17 or 19 or the polymer conjugate or pharmaceutical composition of Claim 18 or 19, wherein the disease or condition is selected from lung tumor, breast tumor, colon tumor, ovarian tumor, prostate tumor, melanoma tumor, lung cancer, breast cancer, colon cancer, ovarian cancer, prostate cancer, and melanoma.

21. Use of the polymer conjugate of any one of Claims 1 to 14 or the pharmaceutical composition of Claim 15 in the preparation of a medicament for imaging a portion of tissue.

22. A polymer conjugate of any one of Claims 1 to 14 or the pharmaceutical composition of Claim 15 for use in imaging a portion of tissue.

23. The use of Claim 21, or the polymer conjugate or pharmaceutical composition of Claim 22, wherein the tissue is from a tumor selected from the group consisting of lung tumor, breast tumor, colon tumor, and ovarian tumor.

24. The use of any one of Claims 17, 19-21, and 23 or the polymer conjugate or pharmaceutical composition of any of Claims 18-20 and 22-23, wherein the polymer conjugate is administered intravenously.

## Patentansprüche

1. Polymerkonjugat, das wenigstens eine sich wiederholende Einheit umfasst, die aus Formel I, Formel II und Formel III ausgewählt ist: 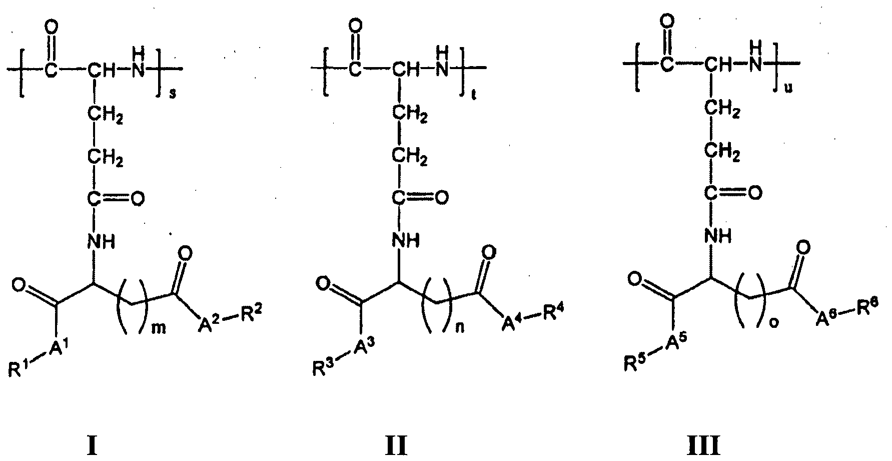 wobei:
A¹, A², A³, A⁴, A⁵ und A⁶ jeweils, unabhängig voneinander, Sauerstoff oder NR⁷ ist, wobei R⁷ Wasserstoff oder ein C₁₋₄-Alkyl ist,
R¹, R², R³, R⁴, R⁵ und R⁶ jeweils, unabhängig voneinander, aus der Gruppe ausgewählt ist, die besteht aus Wasserstoff, einer C₁₋₁₀-Alkyl-Gruppe, einer C₆₋₂₀-ArylGruppe, einer Ammoniumgruppe, einem Alkalimetall, einem mehrzähnigen Liganden, einem Vorläufer eines mehrzähnigen Liganden mit geschützten Sauerstoffatomen, einer Gruppe, die Platin umfasst, einer Gruppe, die ein Arzneimittel umfasst, einer Gruppe, die ein Targeting-Agens umfasst, einer Gruppe, die ein Agens für eine optische Bildgebung umfasst, einer Gruppe, die ein Agens für eine Magnetresonanz-Bildgebung umfasst, und einer Gruppe, die einen Stabilisator umfasst,
wobei der mehrzähnige Ligand umfasst: 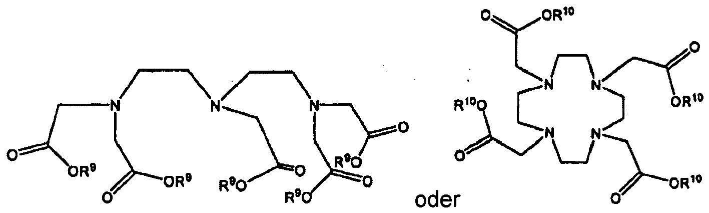 wobei jedes R⁹, unabhängig voneinander, Wasserstoff, Ammonium oder ein Alkalimetall ist,
und
wobei jedes R¹⁰, unabhängig voneinander, Wasserstoff, Ammonium oder ein Alkalimetall ist,
wobei der Vorläufer des mehrzähnigen Liganden mit geschützten Sauerstoffatomen umfasst: 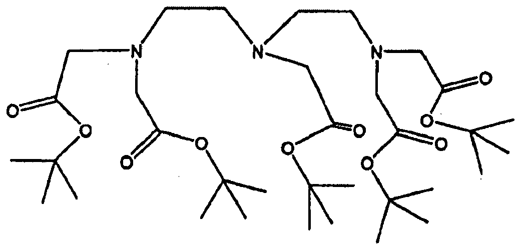 wobei die Gruppe, die Platin umfasst, Cisplatin, Carboplatin oder Oxaliplatin umfasst, oder wobei die sich wiederholende Einheit mit der Formel I die folgende Struktur hat: 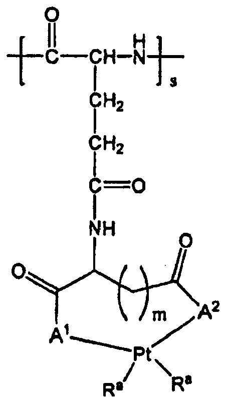 wobei jedes R^{a}, unabhängig voneinander, aus der Gruppe ausgewählt ist, die aus Monoalkylamin, Dialkylamin, Monoarylamin und Diarylamin besteht,
wobei die Gruppe, die ein Arzneimittel umfasst, ein Anti-Krebs-Medikament umfasst,
wobei die Gruppe, die das Targeting-Agens umfasst, aus der Gruppe ausgewählt ist, die aus einem Arginin-Glycin-Aspartat (RGD)-Peptid, Fibronectin, Folat, Galactose, einem Apolipoprotein, Insulin, Transferrin, einem Fibroblast Growth Factor (FGF), einem Epidermal Growth Factor (EGF) und einem Antikörper besteht,
wobei die Gruppe, die ein Agens für eine optische Bildgebung umfasst, aus der Gruppe ausgewählt ist, die aus einem Acridinfarbstoff, einem Cumarinfarbstoff, einem Rhodaminfarbstoff, einem Xanthenfarbstoff, einem Cyaninfarbstoff und einem Pyrenfarbstoff besteht,
wobei die Gruppe, die ein Agens für eine Magnetresonanz-Bildgebung umfasst, umfasst: 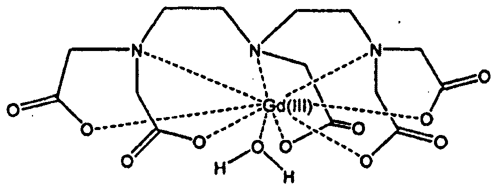 oder 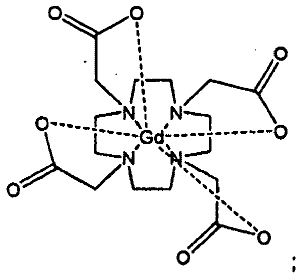 wobei die Gruppe, die einen Stabilisator umfasst, Polyethylenglycol umfasst, wenigstens eines von R¹, R², R³, R⁴, R⁵ und R⁶ eine Gruppe ist, die Platin umfasst,
m, n und o jeweils, unabhängig voneinander, 1 oder 2 ist und
s, t und u jeweils, unabhängig voneinander, 0 oder ≥ 1 ist, wobei s + t + u ≥1 ist.

2. Polymerkonjugat nach Anspruch 1, wobei s 1 oder größer ist und t und u 0 sind.

3. Polymerkonjugat nach irgendeinem der Ansprüche 1 bis 2, wobei die Gruppe, die Platin umfasst, sowohl an A¹ als auch an A² befestigt ist

4. Polymerkonjugat nach Anspruch 3, wobei die sich wiederholende Einheit mit der Formel I die folgende Struktur hat: 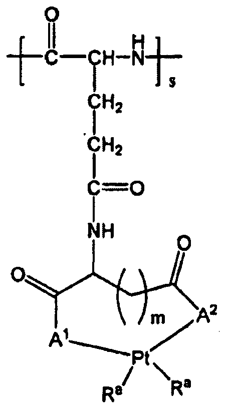 wobei jedes R^{a}, unabhängig voneinander, aus der Gruppe ausgewählt ist, die aus Monoalkylamin, Dialkylamin, Monoarylamin und Diarylamin besteht.

5. Polymerkonjugat nach Anspruch 3, wobei die sich wiederholende Einheit mit der Formel I die folgende Struktur hat:

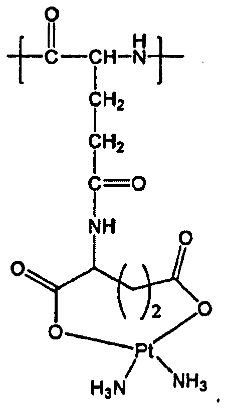

6. Polymerkonjugat nach Anspruch 1, wobei s + t 2 oder größer ist und die Gruppe, die Platin umfasst, an A² und A³ befestigt ist, oder wobei s + t 2 oder größer ist und die Gruppe, die Platin umfasst, an A² und A⁴ befestigt ist, oder wobei s + t 2 oder größer ist und die Gruppe, die Platin umfasst, an A¹ und A³ befestigt ist.

7. Polymerkonjugat nach Anspruch 1 oder Anspruch 6, wobei
s + t 2 oder größer ist, u 0 ist und wenigstens eines von R¹, R², R³ und R⁴ die Gruppe ist, die Platin umfasst, und wenigstens eines von R¹, R², R³ und R⁴ eine Gruppe ist, die das Arzneimittel umfasst, wobei die Gruppe, die Platin umfasst, und die Gruppe, die das Arzneimittel umfasst, nicht die gleichen sind, oder
s + t 2 oder größer ist, u 0 ist und wenigstens eines von R¹, R², R³ und R⁴ eine Gruppe ist, die Platin umfasst, und wenigstens eines von R¹, R², R³ und R⁴ aus der Gruppe ausgewählt ist, die besteht aus dem mehrzähnigen Liganden, dem Vorläufer des mehrzähnigen Liganden mit geschützten Sauerstoffatomen, der Gruppe, die ein Targeting-Agens umfasst, der Gruppe, die ein Agens für eine optische Bildgebung umfasst, der Gruppe, die ein Agens für eine Magnetresonanz-Bildgebung umfasst, und der Gruppe, die einen Stabilisator umfasst.

8. Polymerkonjugat nach Anspruch 1, wobei s + t + u 3 oder größer ist.

9. Polymerkonjugat nach Anspruch 8, wobei R⁵ und R⁶ jeweils, unabhängig voneinander, aus der Gruppe ausgewählt ist, die aus einem Wasserstoff, einer C₁₋₁₀-AlkylGruppe, einer C₆₋₂₀-Aryl-Gruppe, einer Ammoniumgruppe und einem Alkalimetall besteht.

10. Polymerkonjugat nach irgendeinem der Ansprüche 1 to 9, das ferner eine sich wiederholende Einheit mit der Formel IV umfasst: 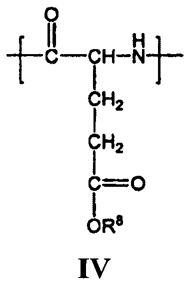 wobei R⁸ Wasserstoff, Ammonium oder ein Alkalimetall ist.

11. Polymerkonjugat nach irgendeinem der Ansprüche 1 bis 10, wobei das Polymerkonjugat eine Gesamtmenge des Platins im Bereich von ungefähr 0,5 % bis ungefähr 50 % (Gewicht/Gewicht) basierend auf dem Verhältnis der Masse des Platins zu derjenigen des Polymerkonjugats umfasst.

12. Polymerkonjugat nach irgendeinem der Ansprüche 1 bis 11, wobei das Anti-Krebs-Medikament aus der Gruppe ausgewählt ist, die aus einem Taxan, einer Camptotheca-Verbindung und Anthracyclin besteht.

13. Polymerkonjugat nach irgendeinem der Ansprüche 1 bis 12, wobei das Anti-Krebs-Medikament ist aus der Gruppe ausgewählt ist, die aus Paclitaxel, Docetaxel, Camptothecin und Doxorubicin besteht.

14. Polymerkonjugat nach irgendeinem der Ansprüche 1 bis 13, das ferner ein Agens umfasst, das PPARγ aktiviert.

15. Pharmazeutische Zusammensetzung, die das Polymerkonjugat nach irgendeinem der Ansprüche 1 bis 14 und wenigstens eine aus einem pharmazeutisch annehmbaren Hilfsstoff, einem Träger und einem Verdünnungsmittel ausgewählte Komponente umfasst.

16. Verfahren zur Herstellung des Polymerkonjugats nach Anspruch 1, das umfasst:
Lösen oder partielles Lösen eines polymeren Reaktionspartners, der eine sich wiederholende Einheit mit der Formel V umfasst, in einem Lösemittel unter Bildung eines gelösten oder partiell gelösten polymeren Reaktionspartners, 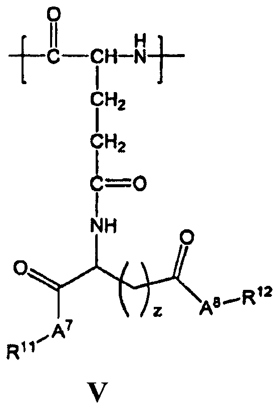 wobei:
z, unabhängig voneinander, 1 oder 2 ist,
A⁷ und A⁸ Sauerstoff sind und
R¹¹ und R¹² jeweils, unabhängig voneinander, aus der Gruppe ausgewählt ist, die aus Wasserstoff, Ammonium und einem Alkalimetall besteht, und
Umsetzen des gelösten oder partiell gelösten polymeren Reaktionspartners mit einem zweiten Reaktionspartner, wobei der zweite Reaktionspartners die Gruppe umfasst, die Platin umfasst.

17. Verwendung des Polymerkonjugats nach irgendeinem der Ansprüche 1 bis 14 oder der pharmazeutischen Zusammensetzung nach Anspruch 15 bei der Zubereitung eines Medikaments für die Behandlung oder Linderung einer Krankheit oder eines Leidens oder bei der Zubereitung eines Medikaments zur Diagnose einer Krankheit oder eines Leidens.

18. Polymerkonjugat nach irgendeinem der Ansprüche 1-14 oder pharmazeutische Zusammensetzung nach Anspruch 15 für die Verwendung bei der Behandlung oder Linderung einer Krankheit oder eines Leidens oder für die Verwendung zur Diagnose einer Krankheit oder eines Leidens.

19. Verwendung nach Anspruch 17 bei der Zubereitung eines Medikaments für die Behandlung oder Linderung einer Krankheit oder eines Leidens oder Polymerkonjugat oder pharmazeutische Zusammensetzung nach Anspruch 18 für die Verwendung bei der Behandlung oder Linderung einer Krankheit oder eines Leidens, ferner umfassend das Verabreichen einer oder mehrerer Verbindung(en), die aus einer Gruppe, die Rosizitaglon umfasst, und einer Gruppe, die Pioglitazon umfasst, ausgewählt ist bzw. sind.

20. Verwendung nach Anspruch 17 oder 19 oder Polymerkonjugat oder pharmazeutische Zusammensetzung nach Anspruch 18 oder 19, wobei die Krankheit oder das Leiden ausgewählt ist aus einem Lungentumor, Brusttumor, Kolontumor, Ovarialtumor, Prostatatumor, Melanomtumor, Lungenkrebs, Brustkrebs, Kolonkrebs, Ovarialkrebs, Prostatakrebs und Melanom.

21. Verwendung des Polymerkonjugats nach irgendeinem der Ansprüche 1 bis 14 oder der pharmazeutischen Zusammensetzung nach Anspruch 15 bei der Zubereitung eines Medikaments für die bildgebende Darstellung eines Teils eines Gewebes.

22. Polymerkonjugat nach irgendeinem der Ansprüche 1 bis 14 oder pharmazeutische Zusammensetzung nach Anspruch 15 für die Verwendung zur bildgebenden Darstellung eines Teils eines Gewebes.

23. Verwendung nach Anspruch 21 oder Polymerkonjugat oder pharmazeutische Zusammensetzung nach Anspruch 22, wobei das Gewebe von einem Tumor stammt, der aus der Gruppe ausgewählt ist, die aus einem Lungentumor, Brusttumor, Kolontumor und einem Ovarialtumor besteht.

24. Verwendung nach irgendeinem der Ansprüche 17, 19-21 und 23 oder Polymerkonjugat oder pharmazeutische Zusammensetzung nach irgendeinem der Ansprüche 18-20 und 22-23, wobei das Polymerkonjugat intravenös verabreicht wird.

## Revendications

1. Conjugué de polymère comprenant au moins un motif récurrent choisi parmi la formule (I), la formule (II) et la formule (III) : 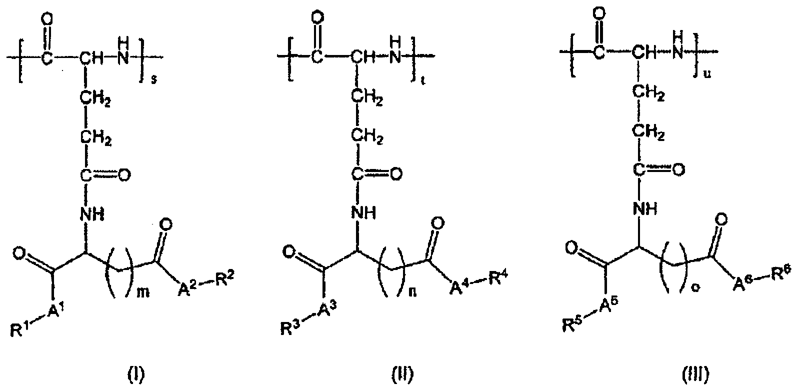 dans lequel :
A¹, A² , A³ , A⁴ , A⁵ et A⁶ sont chacun indépendamment un oxygène ou NR⁷, où R⁷ est un hydrogène ou un alkyle en C₁₋₄ ;
R¹, R², R³, R⁴ , R⁵ et R⁶ sont chacun indépendamment choisis dans le groupe consistant en un hydrogène, un groupe alkyle en C₁₋₁₀, un groupe aryle en C₆₋₂₀, un groupe ammonium, un métal alcalin, un ligand polydentate, un précurseur de ligand polydentate avec des atomes d'oxygène protégés, un groupe qui comprend du platine, un groupe qui comprend un médicament, un groupe qui comprend un agent de ciblage, un groupe qui comprend un agent d'imagerie optique, un groupe qui comprend un agent d'imagerie par résonance magnétique et un groupe qui comprend un agent stabilisant ;
dans lequel le ligand polydentate comprend : 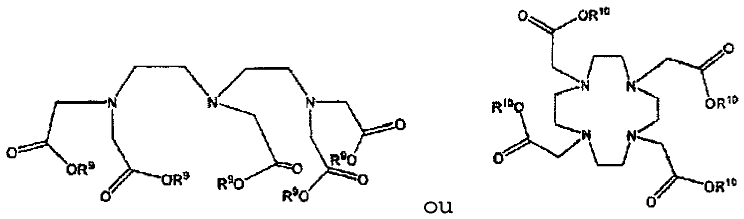 où chaque R⁹ est indépendamment un hydrogène, un ammonium ou un métal alcalin ; et
où chaque R¹⁰ est indépendamment un hydrogène, un ammonium ou un métal alcalin ;
dans lequel le précurseur de ligand polydentate avec des atomes d'oxygène protégés comprend : 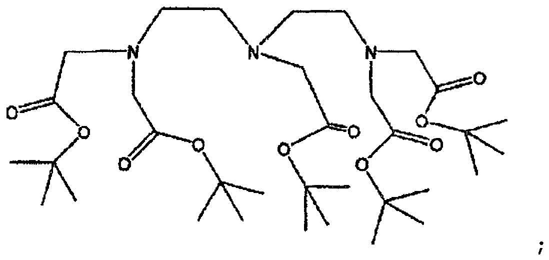 dans lequel le groupe qui comprend du platine comprend le cisplatine, le carboplatine ou l'oxaliplatine, ou dans lequel le motif récurrent de formule (I) a la structure : 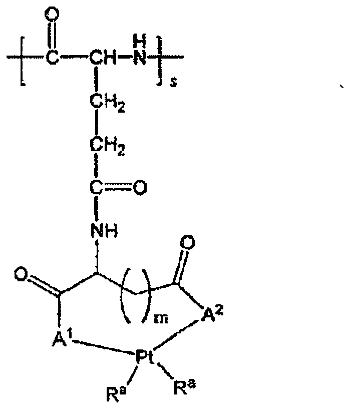 où chaque R^{a} est indépendamment choisi dans le groupe consistant en une monoalkylamine, une dialkylamine, une monoarylamine et une diarylamine ;
dans lequel le groupe qui comprend un médicament comprend un médicament anticancéreux ;
dans lequel le groupe qui comprend l'agent de ciblage est choisi dans le groupe consistant en un peptide arginine-glycine-aspartate (RGD), la fibronectine, le folate, le galactose, une apolipoprotéine, l'insuline, la transferrine, un facteur de croissance des fibroblastes (FGF), un facteur de croissance épidermique (EGF) et un anticorps ;
dans lequel le groupe qui comprend un agent d'imagerie optique est choisi dans le groupe consistant en un colorant acridine, un colorant coumarine, un colorant rhodamine, un colorant xanthène, un colorant cyanine et un colorant pyrène ;
dans lequel le groupe qui comprend un agent d'imagerie par résonance magnétique comprend : 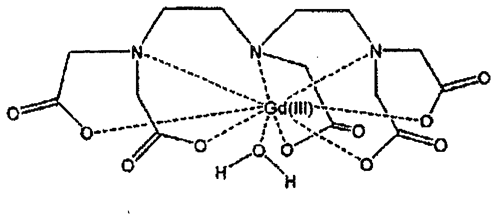 ou
comprend : 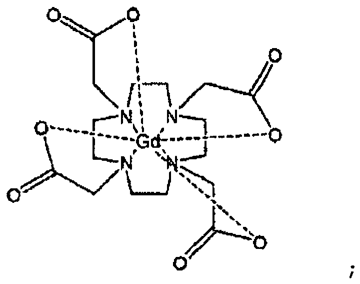 dans lequel le groupe qui comprend un agent stabilisant comprend le poly(éthylène glycol) ;
au moins l'un de R¹, R², R³, R⁴, R⁵ et R⁶ est un groupe qui comprend du platine ;
m, n et o valent chacun indépendamment 1 ou 2 ; et
s, t et u valent chacun indépendamment 0 ou ≥ 1, où s + t + u est ≥ 1.

2. Conjugué de polymère selon la revendication 1, dans lequel s vaut 1 ou plus ; et t et u valent 0.

3. Conjugué de polymère selon l'une quelconque des revendications 1 et 2, dans lequel le groupe qui comprend du platine est attaché à la fois à A¹ et à A².

4. Conjugué de polymère selon la revendication 3, dans lequel le motif récurrent de formule (I) a la structure : 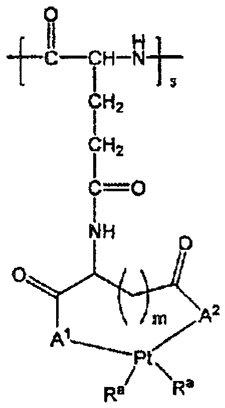 dans laquelle chaque R^{a} est indépendamment choisi dans le groupe consistant en une monoalkylamine, une dialkylamine, une monoarylamine et une diarylamine.

5. Conjugué de polymère selon la revendication 3, dans lequel le motif récurrent de formule (I) a la structure :

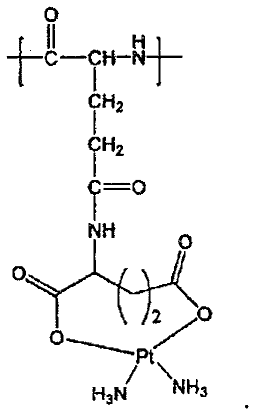

6. Conjugué de polymère selon la revendication 1, dans lequel s + t vaut 2 ou plus ; et le groupe qui comprend du platine est attaché à A² et à A³, ou dans lequel s + t vaut 2 ou plus ; et le groupe qui comprend du platine est attaché à A² et à A⁴, ou dans lequel s + t vaut 2 ou plus ; et le groupe qui comprend du platine est attaché à A¹ et à A³.

7. Conjugué de polymère selon la revendication 1 ou la revendication 6, dans lequel
s + t vaut 2 ou plus ; u vaut 0 ; et au moins l'un de R¹, R ² R³ et R⁴ est le groupe qui comprend du platine, et au moins l'un de R¹, R², R³ et R⁴ est un groupe qui comprend le médicament, dans lequel le groupe qui comprend du platine et le groupe qui comprend le médicament ne sont pas les mêmes ; ou
s + t vaut 2 ou plus ; u vaut 0 ; et au moins l'un de R¹, R², R³ et R⁴ est un groupe qui comprend du platine, et au moins l'un de R¹, R², R³ et R⁴ est choisi dans le groupe consistant en le ligand polydentate, le précurseur de ligand polydentate avec des atomes d'oxygène protégés, le groupe qui comprend un agent de ciblage, le groupe qui comprend un agent d'imagerie optique, le groupe qui comprend un agent d'imagerie par résonance magnétique, et le groupe qui comprend un agent stabilisant.

8. Conjugué de polymère selon la revendication 1, dans lequel s + t + u vaut 3 ou plus.

9. Conjugué de polymère selon la revendication 8, dans lequel R⁵ et R⁶ sont chacun indépendamment choisis dans le groupe consistant en un hydrogène, un groupe alkyle en C₁₋₁₀, un groupe aryle en C₆₋₂₀, un groupe ammonium et un métal alcalin.

10. Conjugué de polymère selon l'une quelconque des revendications 1 à 9, comprenant en outre un motif récurrent de formule (IV) : 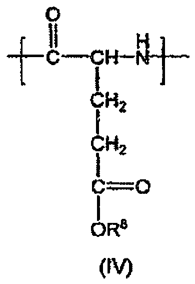 dans laquelle R⁸ est un hydrogène, un ammonium ou un métal alcalin.

11. Conjugué de polymère selon l'une quelconque des revendications 1 à 10, dans lequel le conjugué de polymère comprend une quantité totale de platine dans la plage d'environ 0,5 % à environ 50 % (poids/poids) par rapport au ratio massique entre le platine et le conjugué de polymère.

12. Conjugué de polymère selon l'une quelconque des revendications 1 à 11, dans lequel le médicament anticancéreux est choisi dans le groupe consistant en un taxane, un camptothéca et une anthracycline.

13. Conjugué de polymère selon l'une quelconque des revendications 1 à 12, dans lequel le médicament anticancéreux est choisi dans le groupe consistant en le paclitaxel, le docétaxel, la camptothécine et la doxorubicine.

14. Conjugué de polymère selon l'une quelconque des revendications 1 à 13, comprenant en outre un agent qui active le PPARγ.

15. Composition pharmaceutique comprenant le conjugué de polymère de l'une quelconque des revendications 1 à 14, et au moins un élément choisi parmi un excipient, un vecteur et un diluant pharmaceutiquement acceptables.

16. Procédé de préparation du conjugué de polymère de la revendication 1, comprenant
la dissolution ou dissolution partielle d'un réactif polymérique comprenant un motif récurrent de formule (V) dans un solvant pour former un réactif polymérique dissous ou partiellement dissous ; 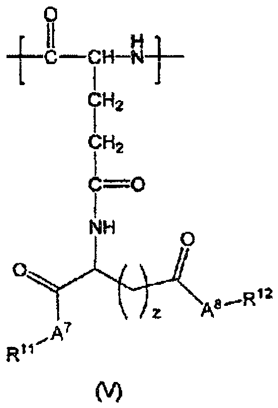 dans laquelle :
z vaut indépendamment 1 ou 2 ;
A⁷ et A⁸ sont un oxygène ; et
R¹¹ et R¹² sont chacun indépendamment choisis dans le groupe consistant en un hydrogène, un ammonium et un métal alcalin ; et
la réaction du réactif polymérique dissous ou partiellement dissous avec un deuxième réactif, dans lequel le deuxième réactif comprend le groupe comprenant du platine.

17. Utilisation du conjugué de polymère de l'une quelconque des revendications 1 à 14 ou de la composition pharmaceutique de la revendication 15, dans la préparation d'un médicament destiné à traiter ou améliorer une maladie ou une affection, ou dans la préparation d'un médicament destiné à diagnostiquer une maladie ou une affection.

18. Conjugué de polymère de l'une quelconque des revendications 1 à 14 ou composition pharmaceutique de la revendication 15, pour une utilisation dans le traitement ou l'amélioration d'une maladie ou d'une affection, ou pour une utilisation dans le diagnostic d'une maladie ou d'une affection.

19. Utilisation selon la revendication 17, dans la préparation d'un médicament destiné à traiter ou améliorer une maladie ou une affection, ou conjugué de polymère ou composition pharmaceutique selon la revendication 18, pour une utilisation dans le traitement ou l'amélioration d'une maladie ou d'une affection, comprenant en outre l'administration d'un ou plusieurs composés choisis dans un groupe qui comprend la rosizitaglone et un groupe qui comprend la pioglitazone.

20. Utilisation selon la revendication 17 ou 19 ou conjugué de polymère ou composition pharmaceutique selon la revendication 18 ou 19, dans laquelle ou lequel la maladie ou l'affection est choisie parmi une tumeur du poumon, une tumeur du sein, une tumeur du côlon, une tumeur de l'ovaire, une tumeur de la prostate, une tumeur de mélanome, un cancer du poumon, un cancer du sein, un cancer du côlon, un cancer de l'ovaire, un cancer de la prostate et un mélanome.

21. Utilisation du conjugué de polymère de l'une quelconque des revendications 1 à 14, ou de la composition pharmaceutique de la revendication 15, dans la préparation d'un médicament destiné à l'imagerie d'une portion de tissu.

22. Conjugué de polymère de l'une quelconque des revendications 1 à 14, ou composition pharmaceutique de la revendication 15, pour une utilisation dans l'imagerie d'une portion de tissu.

23. Utilisation selon la revendication 21, ou conjugué de polymère ou composition pharmaceutique selon la revendication 22, dans laquelle ou lequel le tissu est issu d'une tumeur choisie dans le groupe consistant en une tumeur du poumon, une tumeur du sein, une tumeur du côlon et une tumeur de l'ovaire.

24. Utilisation selon l'une quelconque des revendications 17, 19 à 21 et 23 ou conjugué de polymère ou composition pharmaceutique selon l'une quelconque des revendications 18 à 20, 22 et 23, dans laquelle ou lequel le conjugué de polymère est administré par voie intraveineuse.
